# EUROPÄISCHE PATENTSCHRIFT

(11) **EP 2 157 903 B1**
(45) Veröffentlichungstag und Bekanntmachung des Hinweises auf die Patenterteilung: **12.12.2012**
(21) Anmeldenummer: 08756820.0
(22) Anmeldetag: 12.06.2008
(51) Int. Cl.: A61B 3/113, H04H 60/33, A61B 5/16

(54) **VERFAHREN ZUR WAHRNEHMUNGSMESSUNG**
METHOD FOR PERCEPTION MEASUREMENT
PROCEDE DE MESURE DE PERCEPTION

(30) Priorität: 12.06.2007 AT 9112007
(43) Veröffentlichungstag der Anmeldung: 03.03.2010
(73) Patentinhaber: Pfleger, Ernst, 1010 Wien (AT); Pfleger, Christoph, 1010 Wien (AT)
(72) Erfinder: Pfleger, Ernst, 1010 Wien (AT); Pfleger, Christoph, 1010 Wien (AT)
(74) Vertreter: Gibler & Poth Patentanwälte OG
(86) Internationale Anmeldenummer: PCT/AT2008/000210
(87) Internationale Veröffentlichungsnummer: WO 2008/151346

(56) Entgegenhaltungen:
- EP-A- 1 219 243
- WO-A-03/024319
- WO-A-2006/024129
- US-A- 6 090 051
- US-A- 6 120 461
- US-A1- 2005 073 136
- KRUPINSKI ET AL: "Eye-movement study and human performance using telepathology virtual slides. Implications for medical education and differences with experience" HUMAN PATHOLOGY, SAUNDERS, PHILADELPHIA, PA, US, Bd. 37, Nr. 12, 25. November 2006 (2006-11-25), Seiten 1543-1556, XP005742700 ISSN: 0046-8177
- SODHI M ET AL: "On road driver eye movement tracking using head-mounted devices" PROCEEDINGS ETRA 2002 EYE TRACKING RESEARCH & APPLICATIONS SYMPOSIUM. NEW ORLEANS, LA, MARCH 25 - 27, 2002, NEW YORK, NY : ACM, US, 1. Januar 2002 (2002-01-01), Seiten 61-68, XP002987640 ISBN: 978-1-58113-467-4

## Beschreibung

Die Erfindung betrifft ein Verfahren zur Wahrnehmungsmessung, insbesondere zur Messung visueller Aufmerksamkeit, gemäß dem Oberbegriff des Patentanspruches 1.

Es ist bekannt mittels sog. Blickerfassungssysteme zu ermitteln, auf welchen Bereich bzw. Ort des Blickfelds der Blick eines Betrachters ruht. Derartige Blickerfassungssysteme ermitteln dabei sog. Sichtkoordinaten, daher Koordinaten im Blickfeld des Betrachters, auf welche der Blick des Betrachters gerichtet ist. Ein besonders herausragendes und exaktes Verfahren zur Ermittlung dieser Sichtkoordinaten ist etwa aus der EP 1 300 018 B1 bekannt. Aufgabe der Erfindung ist es daher ein Verfahren zur Wahrnehmungsmessung, insbesondere zur Messung visueller Aufmerksamkeit der eingangs genannten Art anzugeben, mit welchem möglichst exakt die visuelle Aufmerksamkeit für bestimmte Bereiche der Umgebung gemessen werden kann.

Erfindungsgemäß wird dies durch die Merkmale des Patentanspruches 1 erreicht.

Dadurch können wissenschaftlich fundiert und begründet die Wahrnehmung der Umgebung durch einen Probanden bzw. dessen Aufmerksamkeit für bestimmte Bereiche der Umgebung gemessen werden. Es können etwa bei einer vorgebbaren Umgebung die Bereiche, welche durch den Probanden sicher und bewusst wahrgenommen werden, sowie die Bereiche einer ungeordneten und lediglich beiläufigen Blickzuwendung exakt bestimmt werden. So kann die Qualität einer Umgebung, etwa einer Arbeitsplatzumgebung, vor allem in sicherheitsrelevanten bzw. gefährlichen Bereichen, beispielsweise einer Straße, vor allem von Kurven, Baustellenbereichen und/oder Ortsdurchfahrten, einer Benutzerbildschirmoberfläche, von Schaltzentralen, einem Maschinenbedienfeld, etwa der Cockpitgestaltung von Kraftfahrzeugen und Luftfahrzeugen, und/oder eines Werbemediums, etwa einer Bild-und/oder Textanzeige bzw. eines so. Werbespots, bewertet bzw. gemessen werden. Dadurch können vor allem Bereiche der Umgebung bzw. der Umwelt, welche eine akute Gefahr für Leib und Leben darstellen hinsichtlich deren Wahrnehmbarkeitsgrad bewertet bzw. gemessen, und im Hinblick auf eine Verbesserung der Aufnahme wichtiger Informationen umgestaltet werden. Etwa kann der Verlauf einer Straße bereits bei deren Planung im Hinblick auf geringe Umfallrisiken optimiert werden, wichtige Verkehrszeichen, etwa Stopp-Schilder, können gezielt und wissenschaftlich fundiert an Orten aufgestellt werden, an welchen diesen ein hohes Maß an Aufmerksamkeit durch den Straßenverkehrsteilnehmer zuteil wird. Arbeitsplatzumgebungen können gezielt derart gestaltet werden, dass wesentliche und sicherheitsrelevante Kontrollelemente, Anzeigen und Bedienelemente die gezielte Wahrnehmung durch den Benutzer fördern; und Werbemittel können auf die gezielte Wahrnehmung des Betrachters abgestimmt werden.

Dokument US 2005073136 offenbart ein Verfahren gemäß das Oberbegriffs des Anspruchs 1.

Die Unteransprüche, welche ebenso wie der Patentanspruch 1 gleichzeitig einen Teil der Beschreibung bilden, betreffen weitere vorteilhafte Ausgestaltungen der Erfindung.

Die Erfindung wird unter Bezugnahme auf die beigeschlossenen Zeichnungen, in welchen lediglich bevorzugte Ausführungsformen beispielhaft dargestellt sind, näher beschrieben. Dabei zeigt:
Fig. 1 ein Blockschaltbild einer ersten Ausführungsform der Erfindung;
Fig. 2 ein menschliches Auge in geschnittener Darstellung;
Fig. 3 ein Blockschaltbild einer zweiten Ausführungsform der Erfindung;
Fig. 4 ein Blockschaltbild einer dritten Ausführungsform der Erfindung;
Fig. 5 ein Blockschaltbild einer vierten Ausführungsform der Erfindung;
Fig. 6 eine schematische Darstellung des Blickverhaltens bei einer Fixation;
Fig. 7 eine schematische Darstellung des Blickverhaltens bei einer Abfolge einer ersten Fixation, einer Sakkade und einer zweiten Fixation;
Fig. 8 eine bevorzugte Ausführungsform einer Ausgabe des ersten Relativabstandes;
Fig. 9 eine erste bevorzugte Ausgabe eines Blickfeldvideos mit einem ersten und einem zweiten Kreis;
Fig. 10 eine zweite bevorzugte Ausgabe eines Blickfeldvideos mit einem dritten Kreis;
Fig. 11 eine dritte bevorzugte Ausgabe eines Blickfeldvideos mit einem vierten Kreis;
Fig. 12 eine bevorzugte Benutzeroberfläche einer bevorzugten computerimplementierten Ausführungsform der Erfindung;
Fig. 13 eine erste bevorzugte Ausgabe der Häufigkeit der ermittelten Fixationen in Abhängigkeit des Fixationswinkels;
Fig. 14 eine erste bevorzugte Ausgabe der Häufigkeit der ermittelten Sakkaden in Abhängigkeit des Sakkadenwinkels;
Fig. 15 eine erste bevorzugte Ausgabe der Häufigkeit der Fixationen in Abhängigkeit des variablen Fixationskriteriums als Kurvenschar mit konstanter erster Zeitdauer;
Fig. 16 eine vierte bevorzugte Ausgabe eines Blickfeldvideos;
Fig. 17 eine fünfte bevorzugte Ausgabe eines Blickfeldvideos;
Fig. 18 eine sechste bevorzugte Ausgabe eines Blickfeldvideos;
Fig. 19 eine siebente bevorzugte Ausgabe eines Blickfeldvideos;
Fig. 20 eine achte bevorzugte Ausgabe eines Blickfeldvideos;
Fig. 21 eine schematische Darstellung des mit dem Kopf einer Testperson verbundenen Teils eines Blickerfassungssystems;
Fig. 22 eine schematische Darstellung eines Augenbildes;
Fig. 23 eine schematische Darstellung eines Blickfeldbildes;
Fig. 24 eine neunte bevorzugte Ausgabe eines Blickfeldvideos;
Fig. 25 eine zehnte bevorzugte Ausgabe eines Blickfeldvideos;
Fig. 26 eine elfte bevorzugte Ausgabe eines Blickfeldvideos;
Fig. 27 eine zwölfte bevorzugte Ausgabe eines Blickfeldvideos;
Fig. 28 eine bevorzugte Ausgabemaske in einer ersten Ansicht;
Fig. 29 eine bevorzugte Ausgabemaske in einer zweiten Ansicht;
Fig. 30 eine erste bevorzugte Ausgabe der Häufigkeit der ermittelten Fixationen in Abhängigkeit der Fixationsdauer;
Fig. 31 eine erste bevorzugte Ausgabe der Häufigkeit der ermittelten Sakkaden in Abhängigkeit der Sakkadendauer;
Fig. 32 eine erste bevorzugte Ausgabe der Häufigkeit der ermittelten Lidschläge in Abhängigkeit der Lidschlagdauer;
Fig. 33 und Fig. 34 ein erstes Beispiel einer bevorzugten Ausgabemaske eines bevorzugten Analysewerkzeugs; und
Fig. 35 und Fig. 36 ein zweites Beispiel der bevorzugten Ausgabemaske des bevorzugten Analysewerkzeuges.

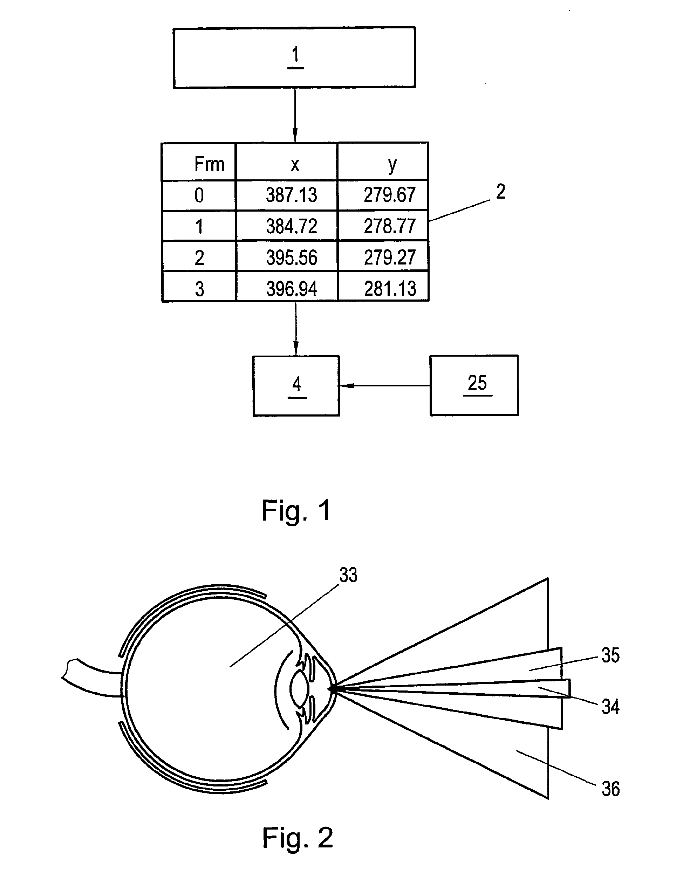

Die Fig. 1, 3, 4 und 5 zeigen jeweils Blockschaltbilder bevorzugter Ausführungsformen eines Verfahrens zur Wahrnehmungsmessung, insbesondere zur Messung visueller Aufmerksamkeit, wobei - insbesondere durch ein Blickerfassungssystem ermittelte - wenigstens erste, einem ersten Blickfeldbild zugeordnete, Sichtkoordinaten eines ersten Blickpunktes 37 und wenigstens zweite, einem zweiten Blickfeldbild zugeordnete, Sichtkoordinaten eines zweiten Blickpunktes 38 verarbeitet werden, wobei das zweite Blickfeldbild nachfolgend dem ersten Blickfeldbild aufgenommen wurde, und die zweiten Sichtkoordinaten des zweiten Blickpunktes 38 mit den ersten Sichtkoordinaten des ersten Blickpunktes 37 in einer Vergleichsvorrichtung auf die Erfüllung wenigstens eines ersten vorgebbaren Fixationskriteriums 25 geprüft werden, und dass die ersten und die zweiten Blickpunkte 37, 38 bei Erfüllung des ersten Fixationskriteriums 25 einer der geordneten Wahrnehmung zuzuordnenden ersten Fixation 48 zugeordnet und gekennzeichnet werden, und dass die ersten und die zweiten Blickpunkte 37, 38 bei Nichterfüllung des ersten Fixationskriteriums 25 einer der ungeordneten Wahrnehmung zuzuordnenden ersten Sakkade zugeordnet und gekennzeichnet werden.

Dadurch können wissenschaftlich fundiert und begründet die Wahrnehmung der Umgebung durch einen Probanden bzw. dessen Aufmerksamkeit für bestimmte Bereiche der Umgebung gemessen werden. Es können etwa bei einer vorgebbaren Umgebung die Bereiche, welche durch den Probanden sicher und bewusst wahrgenommen werden, sowie die Bereiche einer ungeordneten und lediglich beiläufigen Blickzuwendung exakt bestimmt werden. So kann die Qualität einer Umgebung, etwa einer Arbeitsplatzumgebung, vor allem in sicherheitsrelevanten bzw. gefährlichen Bereichen, beispielsweise einer Straße, vor allem von Kurven, Baustellenbereichen und/oder Ortsdurchfahrten, einer Benutzerbildschirmoberfläche, von Schaltzentralen, einem Maschinenbedienfeld, etwa der Cockpitgestaltung von Kraftfahrzeugen und Luftfahrzeugen, und/oder eines Werbemediums, etwa einer Bild- und/oder Textanzeige bzw. eines so. Werbespots, bewertet bzw. gemessen werden. Dadurch können vor allem Bereiche der Umgebung bzw. der Umwelt, welche eine akute Gefahr für Leib und Leben darstellen hinsichtlich deren Wahrnehmbarkeitsgrad bewertet bzw. gemessen, und im Hinblick auf eine Verbesserung der Aufnahme wichtiger Informationen umgestaltet werden. Etwa kann der Verlauf einer Straße bereits bei deren Planung im Hinblick auf geringe Umfallrisiken optimiert werden, wichtige Verkehrszeichen, etwa Stopp-Schilder, können gezielt und wissenschaftlich fundiert an Orten aufgestellt werden, an welchen diesen ein hohes Maß an Aufmerksamkeit durch den Straßenverkehrsteilnehmer zuteil wird. Arbeitsplatzumgebungen können gezielt derart gestaltet werden, dass wesentliche und sicherheitsrelevante Kontrollelemente, Anzeigen und Bedienelemente die gezielte Wahrnehmung durch den Benutzer fördern; und Werbemittel können auf die gezielte Wahrnehmung des Betrachters abgestimmt werden.

Die in der gegenständlichen Ausfertigung gewählten Begrifflichkeiten hinsichtlich erster, zweiter, dritter usw. Sichtkoordinaten, Blickpunkte, Fixationen, Sakkaden, Kreise, Fixations- und/oder Sakkadenwinkel und dergleichen sind bevorzugt in keinem Falle als Einschränkung des Verfahrensablaufes auf lediglich zwei derartig gekennzeichnete Merkmale bzw. nur einen einzelnen Verfahrensdurchlauf bzw. Verfahrensablauf zu verstehen, sondern als Beschreibung eines einzelnen Ablaufs eines vorgebbar oft wiederholbaren Verfahrens.

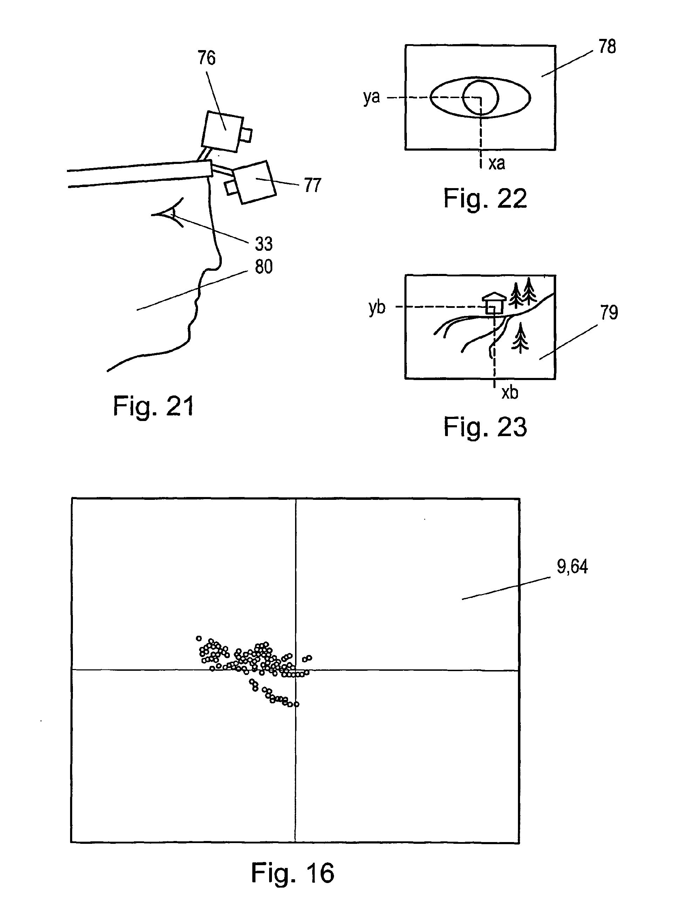

Bei den erfindungsgemäßen Verfahren werden Daten verarbeitet welche mittels eines sog. Blickerfassungssystems ermittelt werden. Ein solches Blickerfassungssystem ist etwa schematisch in der Fig. 21 dargestellt. Ein besonders herausragendes Blickerfassungssystem ist etwa in der EP 1 300 108 A1 beschrieben. Ein solches Blickerfassungssystem, welches im Folgenden kurz beschrieben wird, arbeitet etwa nach einem Verfahren zur Erfassung, Auswertung und Analyse von Blicksequenzen einer Testperson mittels eines Blickerfassungssystems, wobei das Blickfeld der Testperson durch eine nach vorne gerichtete und starr mit dem Kopf 80 der Testperson verbundene erste Kamera 76 erfasst und in einem Blickfeldvideo aufgezeichnet wird, die Bewegung der Pupille der Testperson durch eine zweite Kamera 77, die ebenfalls starr mit dem Kopf 80 der Testperson verbunden ist, erfasst und in einem Augenvideo aufgezeichnet wird, und das Augenvideo und das Blickfeldvideo 9 auf ein Videosystem aufgezeichnet und zeitlich synchronisiert werden, wobei für jedes Einzelbild des Augenvideos, daher für jedes Augenbild 78 die Pupillenkoordinaten xa,ya ermittelt werden, die Korrelationsfunktion K zwischen Pupillenkoordinaten xa,ya auf dem Augenvideo und den Koordinaten xb,yb des entsprechenden Blickpunktes B, d.h. des Punktes, den die Testperson fixiert, auf dem Blickfeldbild 79 des Blickfeldvideos 9 ermittelt wird, und nach erfolgter Ermittlung der Korrelationsfunktion K für jedes Einzelbild aus den Pupillenkoordinaten xa,ya auf dem Augenvideo die Koordinaten xb,yb des entsprechenden Blickpunktes B auf dem Blickfeldvideo extrapoliert werden, wobei zur Ermittlung der Pupillenkoordinaten xa,ya für jedes Einzelbild des Augenvideos mit einem Bilderkennungsprogramm automatisch die Kontraste der Pupille zum Umfeld registriert werden, alle Punkte des Einzelbilds, die dunkler als ein eingestellter Dunkelheitsgrad sind, gesucht werden, mit diesen Punkten eine der Pupille entsprechende Dunkelfläche voll erfasst und abgegrenzt wird, und der dem Pupillenmittelpunkt mit den Pupillenkoordinaten xa,ya entsprechende Schwerpunkt der Dunkelfläche ermittelt wird. Bevorzugt kann dabei vorgesehen sein, dass eine vorgebbare Anzahl von Punkten am Rand der Pupille ausgewählt werden, welche aufgrund deren Kontrasts zur Umgebung besonders gut und sicher identifiziert werden können, und dass diese Punkte als Teil einer Ellipse angenommen werden, woraufhin der Schwerpunkt bzw. der Mittelpunkt einer Ellipse berechnet wird, auf deren Umfang auch die vorgebbare Anzahl an Punkten liegt. Dadurch wird eine besonders hohe Genauigkeit erreicht, welche weit über den aus dem Stand der Technik bekannten Blickerfassungssystemen liegt. Dadurch bleiben Fehler, wie diese etwa durch Reflexionen am Auge entstehen können, ohne Einfluss auf das Messergebnis. Fig.22 zeigt ein schematisches Beispiel für ein Augenbild 78 eines Augenvideos mit den Pupillenkoordinaten xa,ya. Fig. 23 zeigt ein schematisches Beispiel für ein Blickfeldbild 79 mit den Koordinaten xb,yb des ersten Blickpunktes 37. Die Aufgabe von Blickerfassungssystemen ist es, mit möglichst großer Genauigkeit darzustellen, auf welchen Punkt des Blickfeldes eine Testperson blickt, d.h. auf welchen genauen Punkt das Interesse bzw. die Aufmerksamkeit der Testperson gerichtet ist.

Dabei wird einerseits das Blickfeld durch eine nach vorne gerichtete und starr mit dem Kopf 80 der Testperson verbundenen ersten Kamera 76 erfasst. Weiters wird die Bewegung der Pupille der Testperson durch eine zweite Kamera 77, die ebenfalls starr mit dem Kopf 80 verbunden ist, erfasst. Starr verbunden bedeutet in diesem Zusammenhang, dass beide Kameras 76, 77 so mit dem Kopf 80 der Testperson verbunden sind, dass sie sich mit diesem bewegen bzw. allen Bewegungen folgen, wobei aber die Bewegungsfreiheit des Kopfes und der Augen in keiner Weise eingeschränkt wird. Aus der Auswertung dieser beiden Aufnahmen ist es möglich, mit großer Genauigkeit anzugeben, auf welchen Punkt die Testperson gerade blickt. Es sind dadurch Aussagen über Blickzuwendungen, Blickbindungen und Blickabsenzen möglich.

Solche Blickerfassungssysteme werden bevorzugt im Sicherheitsbereich, insbesondere im Bereich der Unfallforschung, aber auch im Bereich der Werbung, des Sports oder bei anderen humanphysiologischen Untersuchungen eingesetzt.

Insgesamt stellt die Blickverhaltensforschung einen wesentlichen Baustein zur Erforschung von physiologischen Unfallursachen dar. Beispielsweise können durch umfassende Blickuntersuchungen neue Erkenntnisse für die Unfallaufklärung und Unfallrekonstruktion hinsichtlich menschlicher Leistungsgrenzen gefunden werden.

So können besonders gefährliche Stellen im Straßenverkehr mit Blickerfassungssystemen untersucht werden. Eine mit einem solchen Blickerfassungssystem ausgestattete Testperson durchfährt dabei die gefährliche Stelle, wobei ihr Blickverhalten aufgezeichnet wird. Die Summe der dabei analysierten Blicke wird im folgenden mit Blicksequenz bezeichnet. Aus der Analyse des Blickverhaltens kann nachvollzogen werden, welche Wegweiser oder Signaltafeln aufgrund ihrer ungünstigen Platzierung nicht genügend Beachtung finden, oder wo besonders uneinsichtige bzw. wenig beachtete Stellen in einer Kreuzung liegen. Im Bereich der Arbeitssicherheit, z.B. auf Baustellen kann untersucht werden, welche Gefahrenquellen erst sehr spät von der Testperson erfasst werden, und welche Sicherheitsvorkehrungen hier notwendig wären. Ein weiteres wichtiges Anwendungsgebiet von Blickerfassungssystemen liegt in der Analyse von Werbeplakaten oder Werbespots. Auch hier kann sehr genau erfasst werden, welche Botschaften, Texte Logos etc. von der Testperson wie lange und in welcher Reihenfolge fixiert werden.

Fig. 21 zeigt einen Teil eines Blickerfassungssystems zur Durchführung eines bevorzugten Verfahrens zur Ermittlung der Blickpunkte bzw. der Sichtkoordinaten (SKO). Das Blickfeld der Testperson wird durch eine nach vorne gerichtete und starr mit dem Kopf 80 der Testperson verbundene erste Kamera 76 erfasst. Diese erste Kamera 76 gibt somit ein ungefähres Bild der Blickrichtung der Testperson, welche rein durch die Position des Kopfes 80 definiert ist. Die erste Kamera 76 kann z.B. durch eine CCD-Farbkamera realisiert sein, die einen Großteil des Blickfeldes der Testperson aufzeichnet.

Vorzugsweise kann die erste Kamera 76 und/oder die zweite Kamera 77 zusätzlich mittels Software gesteuert werden und so den unterschiedlichsten äußeren Einsatzbedingungen angepasst werden. Dies stellt sicher, dass durch die direkte Aufnahme der Pupille keinerlei Verzerrung des Pupillenbildes gegeben ist, bzw. durch die direkte Nähe zum Auge 33 eine große Abbildung vorhanden ist und die Einrichtung insgesamt kleiner gehalten werden kann. Bisherige Verfahren stellen sowohl durch die Größe, als auch durch die generelle schlechtere Zuordnung des Pupillenpunkts erhebliche Ursachen für Unschärfen dar. Dies bedeutet nicht nur Erschwerungen im Gewicht des Blickerfassungssystems, sondern auch generelle Einschränkungen im Blickverhalten der Testperson, welche durch das erfindungsgemäße Verfahren vermieden werden. Daher kann das erfindungsgemäße Blickerfassungssystem auch von Probanden mit unterschiedlichen Kleidungen und Schutzmaßnahmen wie z.B. Helm, ohne Einschränkungen verwendet werden. Es können daher auch sehr leicht unterschiedliche Kameras 76, 77 mit verschiedenen Objektiven je nach Versuchsanforderungen Anwendung finden.

Die, vorzugsweise qualitativ hochwertigen, Kameras, die im bevorzugten System eingesetzt werden, sind vorzugsweise mit einer Control Unit ausgestattet, die es ermöglicht automatischen Weißabgleich, Farbbalance und Belichtung vorzunehmen. Diese Werte sind bevorzugt auch manuell einstellbar. Durch diese Control Unit wird es ermöglicht die Bildqualität den Versuchsbedingungen optimal anzupassen. Hiermit ist eine sehr hohe Bildqualität zur weiteren Analyse sichergestellt. Weiters besteht vorzugsweise die Option, den Bildausschnitt als digitalen Zoom elektronisch zu vergrößern. Andere Einstellmöglichkeiten haben in der Regel nur bedingten Einfluss auf das generierte Bild.

Die Bewegung der Pupille der Testperson wird durch eine zweite Kamera 77 erfasst, die ebenfalls starr mit dem Kopf 80 der Testperson verbunden ist, und auf eines der beiden Augen 33 der Testperson gerichtet ist. Die zweite Kamera 77 kann beispielsweise durch eine s/w CCD-Kamera realisiert sein und die Augenbewegungen des rechten Auges registrieren. Die Erfassung der Pupillenposition durch die zweite Kamera 77 erfolgt bei den in den Figuren dargestellten Blickerfassungssystemen unmittelbar, wobei die zweite Kamera 77 direkt auf das Auge 33 der Testperson gerichtet ist. Die Erfassung der Pupillenposition kann aber auch über optische Umlenksysteme wie Spiegel oder Glasfaserkabel erfolgen, mit denen das Bild vom Auge 33 an die zweite Kamera 77 umgelenkt wird.

Die beiden Kameras 76, 77 sind beispielsweise auf einem Helm oder einer Brille oder einer ähnlichen vorzugsweise leicht auf und abnehmbaren Trägereinrichtung aufgebracht, die starr mit dem Kopf 80 der Testperson verbunden sind. Unter starr verbunden ist, wie oben bereite erläutert, zu verstehen, dass die Trägereinrichtung und beide Kameras 76, 77 allen Bewegungen des Kopfes 80 folgen, wobei aber die Bewegungsfreiheit des Kopfes 80 und der Augen 33 in keiner Weise eingeschränkt wird. Die Anbringung der Kameras 76, 77 auf einer Brille als leicht auf und abnehmbare Trägereinrichtung mit direkter Aufzeichnung auf einem mobilen Aufzeichnungsgerät ermöglicht eine besonders große Mobilität der Testperson und erlaubt eine sehr viel größere Versuchsvielfalt als bei herkömmlichen Systemen.

Natürlich ist es auch möglich, mehrere zweite Kameras 77 vorzusehen, beispielsweise um beide Pupillen der Testperson aufzunehmen. Auch können mehrere erste Kameras 76 vorgesehen werden, um das Blickfeld der Testperson vollständig zu erfassen, falls die Brennweite einer einzelnen ersten Kamera 76 hierfür nicht ausreichend ist. Auf diese Weise können einzelne Blicksequenzen erfasst und, wie im folgenden beschrieben, ausgewertet und analysiert werden. Die Bezeichnung Blicksequenz steht dabei für die Summe der jeweils aufgenommenen und analysierten Blicke.

Durch die beiden Kameras 76, 77 erhält man zwei, im folgenden mit Augenvideo und Blickfeldvideo bezeichnete und in Fig. 22 bzw. 23 schematisch dargestellte Videosignale, welche auf ein Videosystem aufgezeichnet werden. Die Bezeichnung Videosystem umfasst hierbei alle Einrichtungen, die geeignet sind, Filmdaten aufzuzeichnen. Es können analoge Filmmaterialien wie auch Videobänder oder digitale Speichermedien wie DVDs oder ähnliche verwendet werden. Auch das Ablegen einzelner Bilder im Speicher eines Computers gilt als Aufzeichnung im Sinne der Erfindung. Es können unterschiedliche analoge oder digitale Filmformate, wie DV, AVI oder MPEG2 verwendet werden. Vorzugsweise werden bei der Verwendung von CCD-Kameras die beiden Bildinformationen auf ein digitales Videosystem, beispielsweise auf 2 Mini-DV-Rekorder, aufgezeichnet.

Bei einer bevorzugten Ausführungsform wird die Verbindung zwischen den Kameras 76, 77 und dem Videosystem Leitungsgebunden oder über eine Funkstrecke realisiert. Dies ermöglicht die kabellose Übertragung der Videosignale zum Videosystem. Hierdurch wird vorteilhaft das ungehinderte Bewegen der Testperson als Fußgänger, als Radfahrer im freien Gelände oder auch auf bestimmten Arbeitseinsätzen, wie. z.B. auf Gerüsten oder Baustellen ermöglicht.

Es ist wichtig, dass die beiden Videosignale synchronisiert sind, d.h., dass für jedes Einzelbild des Augenvideos das entsprechende Einzelbild des Blickfeldvideos 9 gefunden werden kann und umgekehrt. Die Synchronisierung kann mit einem periodischen Signalgenerator und Time-Code erfolgen. Vorzugsweise wird das Aufzeichnungsverfahren mit einem Tonpuls synchronisiert, der auf den jeweiligen Audiospuren mit aufgezeichnet wird. Dieses Verfahren ermöglicht es, gleichzeitig auch andere externe Geräte, wie z.B. UDS-Datenschreiber, GPS-Systeme o.ä. mit zu synchronisieren um auch weitere technische und medizinische Kenngrößen wie die aktuelle genaue geographische Position oder auch Herz- bzw. Pulsfrequenz, Hautwiderstand, Atemfrequenz etc. der Testperson direkt mit dem Blickverhalten in Abstimmung bringen zu können. Die Synchronisierung ist für die spätere erfindungsgemäße Bearbeitung bzw. Auswertung der beiden Videosignale wichtig.

Bei dem bevorzugten Verfahren werden die genauen Koordinaten (xa,ya) des Pupillenmittelpunktes im Augenvideo durch ein Bilderkennungsprogramm ermittelt. Hierbei werden die Pupillenkoordinaten (xa,ya) für jedes Einzelbild des Augenvideos ermittelt. Die Pupillenkoordinaten (xa,ya) in einem Einzelbild des Augenvideos sind in Fig. 22 skizziert. Die Ermittlung der Pupillenkoordinaten (xa,ya) erfolgt vorzugsweise automatisch mit einem Bilderkennungsprogramm. Hierfür werden für jedes Einzelbild des Augenvideos die Kontraste der Pupille zum Umfeld registriert und alle Punkte des Einzelbilds, die dunkler als ein eingestellter Dunkelheitsgrad sind, gesucht. Mit diesen Punkten wird eine Dunkelfläche voll erfasst und abgegrenzt, und daraufhin automatisch der Schwerpunkt dieser Dunkelfläche ermittelt. Da die Dunkelfläche der Pupille der Testperson entspricht, stellt der Schwerpunkt der Dunkelfläche den Pupillenmittelpunkt dar. Vorzugsweise bietet das Bilderkennungsprogramm Einstellungsvarianten für die entsprechenden Kontraste und den Dunkelheitsgrad, sodass eine besonders hohe Genauigkeitsstufe für alle Einzelbilder erreicht werden kann. Wie vorstehend bereits ausgeführt kann auch zusätzlich vorgesehen sein Punkte am Rand der Pupille auszuwählen, welche aufgrund des Kontrasts zur Umgebung besonders gut und sicher identifiziert werden können, und dass diese Punkte als Teil einer Ellipse angenommen werden, woraufhin der Schwerpunkt bzw. der Mittelpunkt einer Ellipse berechnet wird, auf deren Umfang auch die vorgebbare Anzahl an Punkten liegen. Für jedes Einzelbild kann somit für unterschiedliche Belichtungsverhältnisse der jeweils beste Kontrast in Form einer Grauwertschwelle sichergestellt werden, was insgesamt eine sichere Bestimmung der Pupillenkoordinaten (xa,ya) möglich macht. Die Grauwertschwelle ist jener Wert, der z.B. bei digitalisierter Form zwischen 1 und 256 liegt, und den prozentuellen Anteil von Schwarz bzw. Weiß an einem Bildpunkt definiert. Der höchste erreichbare Wert entspricht einem vollen Schwarz, der niedrigste Wert dem Weiß. Da die Pupille bei der Aufzeichnung voraussichtlich nie den vollen Schwarzwert erreicht, ist ein Wert zu definieren, der - zumindest für dieses Bild - dem real vorhandenen Pupillengrau entspricht. Der Schwellwert scheidet alle Bildpunkte aus, die heller als der definierte Grauwert sind, alle dunkleren Bereiche werden zur Schwerpunktsfindung herangezogen. Drei Parameter ermöglichen es, die Schwellendefinition zu optimieren. Da sich bei den Versuchen innerhalb einer Sequenz die Belichtungsverhältnisse oft sehr stark verändern, ist diese Schwellwertdefinition vorzugsweise auch für jedes Bild einzeln möglich. Alle Einstellungen können entsprechend den hohen Anforderungen für jedes Bild der Sequenz in einer Datei abgelegt werden. Durch das erfindungsgemäße Verfahren ist die besonders hohe Genauigkeit bei der Zuordnung der Pupillenkoordinaten (xa,ya) an das Blickfeld möglich. Die jeweilige Genauigkeitsstufe kann visualisiert werden.

Um eine besonders hohe Genauigkeit der ermittelten Pupillenkoordinaten und somit eine besonders genaue Ermittlung der Blickzuwendung zu erreichen, ist bei einer besonders bevorzugten Ausführungsform der Erfindung vorgesehen, dass zusätzlich eine Korrektur optischen Mängel, insbesondere Objektiventzerrung, eine Perspektivenkorrektur, eine Bildfeldkorrektur und/oder eine Korrektur der sog. Abbildungsfehler, wie etwa der sphärischen Aberration, der chromatischen Aberration, der Dispersion, der Asymmetriefehler (Koma), des Astigmatismus schiefer Bündel (Zweischalenfehler), der Bildfeldwölbung, der Bildkrümmung, der optischen Distorsion, und/oder der monochromatsichen Abbildungsfehler vorgesehen ist.

Zusätzlich kann zur genaueren Lokalisation des Pupillenmittelpunktes ein Infrarotfilter vor der Kamera vorgesehen sein. Durch diesen werden die Kontraste im Augenvideo verstärkt. Der IR Filter hat zwei vorteilhafte Punktionen: Erstens erfolgt die Beleuchtung des Auges 33 mit Infrarot-Leuchtdioden (IR-LED), die auch bei absoluter Dunkelheit gute Kontraste für die Augenkamera und für die weitere Bearbeitung gewährleisten. Der Filter hat die Aufgabe, das von den LED emittierte Licht auf den Kamerachip durchzulassen, alle anderen Spektralbereiche des Lichtes werden entsprechend der Filterdurchlasskurve gedämpft. Zweitens sind die vom Sonnenlicht verursachten Reflexionen auf der Pupille, die sich massiv negativ auf die Schwerpunktsfindung auswirken, hauptsächlich im blauen Spektralbereich vorhanden. Auch hier hat der Filter die Aufgabe die Reflexionen auf der Pupille, die durch das Sonnenlicht verursacht werden, zu reduzieren.

Bei einer weiteren vorteilhaften Ausführung des bevorzugten Verfahrens erfolgt nach der automatischen Ermittlung der Pupillenkoordinaten (xa,ya) zusätzlich eine manuelle Kontrolle. Ein Bearbeiter kann bei einer fehlerhaften automatischen Erkennung (die beispielsweise bei plötzlichen Lichtreflexen auf der Augenoberfläche etc. auftreten) die Bildverarbeitungsparameter manuell ändern. Auch ist es möglich, die Pupillenkoordinaten (xa,ya) unmittelbar zu korrigieren.

Man erhält insgesamt für jedes Einzelbild des Augenvideos die Pupillenkoordinaten (xa,ya) beispielsweise in Form eines kartesischen Wertepaares. Natürlich können auch andere Koordinatensysteme, wie Polarkoordinaten etc. zum Einsatz kommen. Da beide Kameras 76, 77 starr mit dem Kopf 80 der Testperson verbunden sind, entspricht einer bestimmten Position der Pupille bzw. des Pupillenmittelpunktes im Augenvideo stets ein genau definierter Blickpunkt B im Blickfeldvideo 9. Aus dem Augenvideo und dem Blickfeldvideo 9 kann somit berechnet werden, auf welchen Punkt die Testperson genau blickt. Für die Zuordnung der Pupillenkoordinaten (xa,ya) zu den Koordinaten (xb,yb) des entsprechenden Blickpunktes B, d.h. des Punktes, den die Testperson fixiert, auf dem Blickfeldvideo muss zunächst die Korrelationsfunktion K zwischen diesen beiden Koordinatenpaaren (xa,ya) und (xb,yb) ermittelt werden. Die Korrelation zwischen Pupillenkoordinaten (xa,ya) und Blickpunkt B auf dem Blickfeldvideo erfolgt über eine Versuchsserie (Kalibrierung). Hierbei fixiert die Testperson der Reihe nach bestimmte vorgegebene Passpunkte P. Die Korrelationsfunktion K zwischen Pupillenkoordinaten (xa,ya) und den Koordinaten (xb,yb) im Blickfeldvideo wird anhand der hierbei gemessenen Daten erstellt.

Beim bevorzugten Verfahren wird die Korrelationsfunktion K zwischen Pupillenkoordinaten (xa,ya) auf dem Augenvideo und den Koordinaten (xb,yb) des entsprechenden Blickpunktes B auf dem Blickfeldvideo 9 vorzugsweise automatisch ermittelt. Hierfür werden zunächst eine oder mehrere Musterblicksequenzen der Testperson auf einen oder mehrere bestimmte vorgegebene Passpunkte P aufgenommen. Unter Musterblicksequenz ist eine Blicksequenz zu verstehen, welche lediglich für die Kalibrierung aufgenommen wird, und bei der die Testperson auf vorgegebene Passpunkte P blickt. Beispielsweise kann ein bestimmter Passpunkt P auf einer Wand markiert sein. Um einen bestmöglichen Kontrast zu erhalten, kann beispielsweise eine schwarze Markierung auf einer sonst weißen Oberfläche als Passpunkt P gewählt werden. Der Passpunkt P ist in der Regel ein Kreuz oder ein Leuchtpunkt oder ähnliches. Die Testperson wird angewiesen, diesen Passpunkt P zu fixieren, wobei das Blickfeld und das Auge der Testperson durch die beiden Kameras 76, 77 aufgenommen werden. Es können auf diese Weise mehrere Passpunkte P definiert werden. Da der Blickpunkt B auf dem so aufgenommenen Blickfeldvideo der Musterblicksequenz durch den bekannten Passpunkt P gegeben sind, kann in einem nächsten Schritt die Korrelationsfunktion K zwischen den Pupillenkoordinaten (xa,ya) auf dem Augenvideo und den Koordinaten (xb,yb) des entsprechenden Blickpunktes B auf dem Blickfeldvideo ermittelt werden. Hierfür werden für jedes Einzelbild im Augenvideo die Pupillenkoordinaten (xa,ya) im Augenvideo gemäß dem oben beschriebenen Verfahren ermittelt. Weiters werden die Koordinaten (xb,yb) des Passpunktes P im entsprechenden Einzelbild auf dem Blickfeldvideo ermittelt. Dies erfolgt bevorzugt mit einem Bilderkennungsverfahren und/oder einem Mustererkennungsverfahren, welches auf dem Blickfeldvideo die Koordinaten (xb,yb) des durch seinen Kontrast eindeutig erkennbaren Passpunktes P ermittelt. Es ist aber auch möglich, die Koordinaten (xb,yb) des Passpunktes P im Blickfeldvideo jeweils für jedes Einzelbild händisch, beispielsweise durch Mausklickverfahren, zu bestimmen. Dies erlaubt eine Auswertung der Musterblicksequenz auch bei schwierigen Bedingungen im Gelände, bei denen eine automatische Bestimmung der Koordinaten (xb,yb) des Passpunktes P, beispielsweise wegen eines zu uneinheitlichen Hintergrundes, nicht möglich ist.

Somit können die Pupillenkoordinaten (xa,ya) im Einzelbild des Augenvideos den Koordinaten (xb,yb) des Passpunktes P im entsprechenden Einzelbild des Blickfeldvideos zugeordnet werden. Die entsprechenden Koordinaten im Augen- und im Blickfeldvideo werden für jedes Einzelbild der Musterblicksequenz ermittelt und abgespeichert. Aus allen so gewonnenen Datensätzen werden vorzugsweise durch quadratische Regression die Pupillenkoordinaten (xa,ya) auf dem Augenvideo und die Koordinaten (xb,yb) des entsprechenden Blickpunktes B auf dem Blickfeldvideo korreliert, wobei auch andere Verfahren wie lineare Regression oder stochastische Modelle für die Korrelation möglich sind. Man erhält eine Korrelationsfunktion K: (xa,ya) -> (xb,yb), welche einem bestimmten Satz von Pupillenkoordinaten (xa,ya) auf dem Augenvideo eindeutig die entsprechenden Koordinaten (xb,yb) des Blickpunktes B im Blickfeldvideo zuordnet.

Für eine größtmögliche Genauigkeit der Korrelationsfunktion K sollten zumindest 25 unterschiedliche Positionen des Passpunktes P verwendeten werden. Ab etwa 100 unterschiedlichen Passpunktpositionen nimmt die erzielte Genauigkeit kaum mehr zu, sodass eine weitere Erhöhung der Zahl der Passpunktpositionen nicht mehr sinnvoll ist. Vorzugsweise werden somit zwischen 25 und 100 Passpunktpositionen verwendet. Mit der so ermittelten Korrelationsfunktion K können alle weiteren Videosequenzen der gleichen Versuchsserie, d.h. bei denen es keine Änderungen bezüglich der Kamerapositionen auf dem Kopf der Testperson gibt, errechnet werden. Durch die numerische Korrelation der beiden Koordinatenpaare können nichtlineare Zusammenhänge erfasst werden.

Nach der Kalibrierung des Blickerfassungssystems können nun einzelne Blicksequenzen erfasst und analysiert werden. Nach erfolgter Ermittlung der Korrelationsfunktion K werden hierbei für jedes Einzelbild aus den Pupillenkoordinaten (xa,ya) auf dem Augenvideo die Koordinaten (xb,yb) des entsprechenden Blickpunktes B auf dem Blickfeldvideo extrapoliert. Die Zusammenführung des Augenvideos und des Blickfeldvideos 9 in einem Ergebnisvideo erfolgt softwaretechnisch so, dass die errechneten Blickpunkte B als Mittelpunkte der Blickzuwendungen auf dem Blickfeldvideo 9 positioniert werden. Durch die erfindungsgemäße rechnerische Ermittlung der Koordinaten (xb,yb) der Blickpunkte B ist die besonders genaue Darstellung des Mittelpunktes der Blickzuwendung möglich. Der Blickpunkt B kann auf dem Blickfeldvideo 9 genau eingezeichnet werden. Vorzugsweise wird der Blickpunkt B auf dem Blickfeldvideo 9 durch eine visuell gut sichtbare Markierung, beispielsweise durch ein Kreuz, angedeutet.

Mittels eines erfindungsgemäßen Verfahrens kann bestimmt werden, welchen Bereichen der Umgebung einer Testperson bzw. eines Pobanden dessen visuelle Aufmerksamkeit zuteil wird, welche Bereiche der Umgebung tatsächlich durch den Probanden wahrgenommen werden, und welche Bereiche zwar von Blicken gestreift, bzw. überflogen werden, jedoch so kurz bzw. so weit entfernt, dass keine geordnete Wahrnehmung durch den Probanden stattgefunden hat. Daher, dass der Blick des Probanden einen Bereich zwar gestreift hat, der Proband jedoch keine Inhalte dieses Bereichs registriert hat. Bereiche, in denen eine geordnete Wahrnehmung gegeben ist, werden im Weiteren mit dem Begriff Fixation bezeichnet. Bereiche, in denen eine Augenbewegung erfolgte und eine solche geordnete Wahrnehmung nicht gegeben ist, werden im Weiteren mit dem Begriff der Sakkade bezeichnet.

Fig. 2 zeigt eine Schnittdarstellung eines menschlichen Auges 33, wobei Bereiche unterschiedlicher Sehschärfe eingezeichnet sind. Wichtig sind hiebei vor allem der sog. foveale Bereich 34, welcher lediglich in einem eng begrenzten Bereich um eine - nicht eingezeichnete - zentrale Sehachse gegeben ist, und in welchem die höchste Sehschärfe möglich ist, und damit verbunden auch eine geordnete Wahrnehmung visueller Reize. Bisher übliche Definitionen des fovealen Bereichs 34 gehen von einem ersten Blickwinkel 41 von einem Radius von etwa 1° um die Sehachse aus. Wie an weiterer Stelle dieses Dokuments noch ausgeführt werden wird, ist der erste Blickwinkel 41 des fovealen Bereichs 34 jedoch stark objekt- bzw. umgebungsabhängig. Der foveale Bereich 34 wird von dem sog. parafovealen Bereich 35 umgeben, in welchem noch das Erkennen grober Muster möglich ist. Der diesen parafovealen Bereich 35 umgebende sog. periphere Bereich 36 ist lediglich bewegungssensitiv. Ein Erkennen eines Musters bzw. eines Objekts in diesem peripheren Bereich 36 ist nicht möglich.

Bei dem erfindungsgemäßen Verfahren werden wenigstens mittelbar aufeinander folgende Blickpunkte 37, 38 in einer Vergleichsvorrichtung auf das Erfüllen wenigstens eines ersten Fixationskriteriums 25 hin untersucht bzw. verglichen. Bei einer Vergleichsvorrichtung kann es sich um jede geeignete Vorrichtung handeln. Bevorzugt sind Vorrichtungen umfassend elektronische Logikbauteile bzw. sog. Logikgatter vorgesehen, welche einen Vergleich von Eingangsdaten zufolge boolscher Algorithmen ermöglichen. Besonders bevorzugt sind Vorrichtungen, welche derartige elektronische Logikbauteile in integrierter Form aufweisen, insbesondere in Form von Prozessoren, Mikroprozessoren und/oder programmierbaren Logikschaltungen. Besonders bevorzugt kann vorgesehen sein, dass die Vergleichsvorrichtung in einem Computer implementiert ist.

Die Vergleichsvorrichtung verarbeitet sog. Sichtkoordinaten, welche in der weiteren Folge auch kurz als SKO abgekürzt sein können, und welche etwa zufolge einer vorstehend beschriebenen Korrelationsfunktion zwischen einem Blickfeldbild 79 und einem Augenbild 78 ermittelt werden können, wobei auch andere Verfahren bzw. Methoden zur Ermittlung dieser SKO vorgesehen sein können. In Fig. 1 ist mit dem Bezugszeichen 2 eine beispielhafte Liste möglicher SKO für einzelne Blickfeldbilder - als Frm, für Frame, abgekürzt - als kartesische Koordinaten in einer Liste dargestellt.

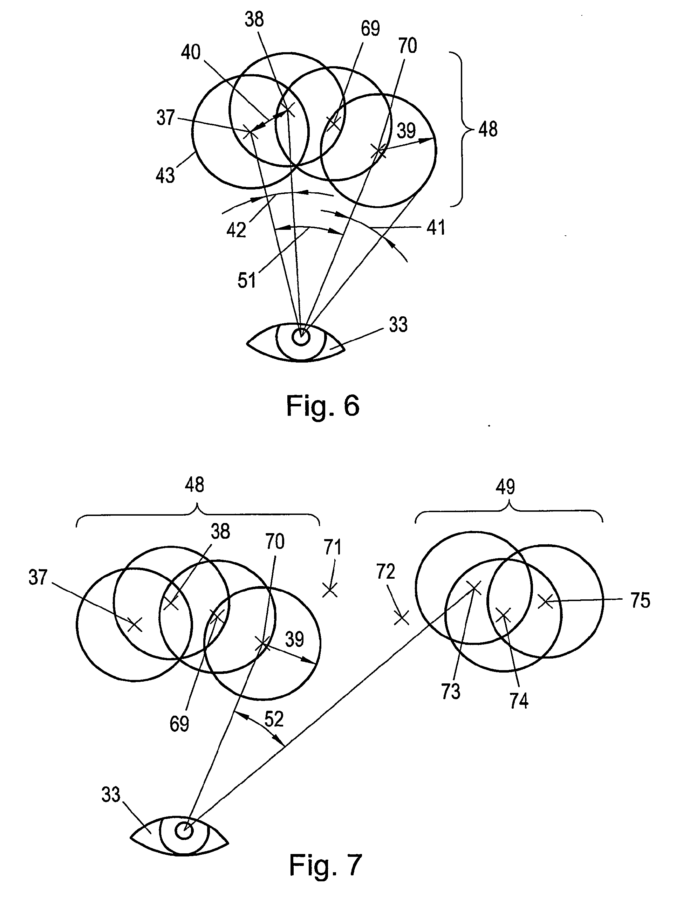

Beim ersten Fixationskriterium 25 kann es sich um jede Art eines Kriteriums handeln, welches eine Unterscheidung zwischen Fixationen und Sakkaden ermöglicht. Bei einer bevorzugten Ausführungsform des erfindungsgemäßen Verfahrens ist vorgesehen, dass das erste Fixationskriterium 25 ein vorgebbarer erster Abstand 39 um den ersten Blickpunkt 37 ist, dass der erste Relativabstand 40 zwischen dem ersten Blickpunkt 37 und dem zweiten Blickpunkt 38 ermittelt wird, und dass wenn der erste Relativabstand 40 geringer als der erste Abstand 39 ist, der erste und der zweite Blickpunkt 37, 38 der ersten Fixation 48 zugeordnet werden, daher solange ein auf einen ersten Blickpunkt 37 folgender zweiter Blickpunkt 38 innerhalb des fovealen Bereichs 34 des ersten Blickpunkts 37 und somit innerhalb des Bereichs geordneter Wahrnehmung des ersten Blickpunkts 37 bleibt, wird die geordnete Wahrnehmung nicht unterbrochen und somit das erste Fixationskriterium 25 weiterhin erfüllt. Es handelt sich daher um eine erste Fixation 48. Bei einer besonders bevorzugten Ausführungsform des erfindungsgemäßen Verfahrens ist vorgesehen, dass der erste Abstand 39 ein erster Blickwinkel 41 ist, welcher vorzugsweise einen dem fovealen Sehen zugeordneten Bereich 34 beschreibt, insbesondere einen Radius zwischen 0,5° und 1,5°, vorzugsweise etwa 1°, und dass der Abstand zwischen dem ersten Blickpunkt 37 und dem zweiten Blickpunkt 38 ein erster Relativwinkel 42 ist. Dadurch ist ausgehend von den mittels eines Blickerfassungssystems ermittelten Sichtkoordinaten eine besonders einfache und genaue Bestimmung von Sakkaden bzw. Fixationen 48, 49 möglich. Fig. 6 zeigt beispielhaft eine erste Fixation 48, welche aus einer Aneinanderfolge von vier Blickpunkten 37, 38, 69, 70 gebildet wird. In Fig. 6 ist dabei auch der erste Abstand 39, der erste Blickwinkel 41, der erste Relativabstand 40 und der erste Relativwinkel 42 eingezeichnet. Um jeden der vier Blickpunkte 37, 38, 69, 70 ist dabei jeweils ein erster Kreis 43 mit dem Radius des ersten Abstandes 39 eingezeichnet, wodurch gut veranschaulicht wird, dass der jeweils nächstfolgende Blickpunkt 38, 69, 70 innerhalb des ersten Kreises 43 mit Radius erster Abstand 39 des vorangehenden Blickpunktes 37, 38, 69 liegt, und somit das bevorzugte erste Fixationskriterium 25 erfüllt ist. Zur Anpassung an unterschiedlich wahrzunehmende Objekte bzw. an unterschiedliche Personen und/oder Gegebenheiten ist bei einer Weiterbildung der Erfindung vorgesehen, dass das erste Fixationskriterium 25, insbesondere der erste Abstand 39 und/oder der erste Blickwinkel 41, vorgebbar ist.

Fig. 7 veranschaulicht eine Blickabfolge, bei welcher nicht sämtliche Blickpunkte 37, 38, 69, 70, 71, 72, 73, 74, 75 für sich dem ersten Fixationskriterium 25 genügen. Die ersten vier Blickpunkte 37, 38, 69, 70 erfüllen jeweils das Fixationskriterium 25 und bilden zusammen die erste Fixation 48, wohingegen die nachfolgenden drei Blickpunkte 71, 72, 73 das erste Fixationskriterium 25 jeweils nicht erfüllen. Erst der vierte, der ersten Fixation 28 nachfolgende Blickpunkt 74 erfüllt das erste Fixationskriterium 25 gegenüber dem dritten, der ersten Fixation 48 nachfolgenden Blickpunkt 73. Der dritte, der ersten Fixation 48 nachfolgende Blickpunkt 73 ist somit der erste Blickpunkt 73 der zweiten Fixation 49, welche aus insgesamt drei Blickpunkten 73, 74, 75 gebildet wird. Bei den Fig. 6 und 7 handelt es vorzugsweise um veranschaulichende Beispiele, können in einer natürlichen Umgebung doch Fixationen 48, 49 mit einer Vielzahl an einzelnen Blickpunkten auftreten. Der Bereich zwischen dem letzten Blickpunkt 70 der ersten Fixation 48 und dem ersten Blickpunkt 73 der zweiten Fixation 49 bildet eine Sakkade, daher einen Bereich ohne Wahrnehmung. Der Winkel zwischen dem letzten Blickpunkt 70 der ersten Fixation 48 und dem ersten Blickpunkt 73 der zweiten Fixation 49 wird als erster Sakkadenwinkel 52 bezeichnet.

Fig. 1 zeigt ein Blockschaltbild eines erfindungsgemäßen Verfahrens, wobei in einem ersten Schritt 1 mittels eines Blickerfassungssystems ein Blickfeldvideo 9 und ein Augenvideo aufgenommen werden. In einem zweiten Verfahrensschritt 2 werden aus dem Blickfeldvideo und dem Augenvideo die SKO ermittelt, welche in einem weiteren Verfahrensschritt 4 in der Vergleichsvorrichtung mit dem festgelegten, gespeicherten, einlesbaren bzw. vorgebbaren ersten Fixationskriterium 25 verglichen werden. Die einzelnen derart einer Sakkade bzw. einer Fixation 48, 49 zugeordneten Blickpunkte 37, 38 können nun zur weiteren Auswertung, Verarbeitung bzw. Darstellung ausgegeben werden. Insbesondere kann vorgesehen sein, dass der erste und der zweite Blickpunkt 37, 38 als der ersten Fixation 48 bzw. der ersten Sakkade zugehörig gekennzeichnet ausgegeben werden.

In der Vergleichsvorrichtung werden zwei - wenigstens mittelbar aufeinander folgende Blickfeldbilder bzw. die diesen zugeordneten SKO verglichen. Bevorzugt ist dabei vorgesehen, dass das zweite Blickfeldbild nach einer vorgebbaren ersten Zeitdauer, insbesondere zwischen 0,005s und 0,1s, vorzugsweise zwischen 0,02s und 0,04s, nachfolgend dem ersten Blickfeldbild aufgenommen wurde. Aufgrund der Bewegungsauflösung des menschlichen Auges 33 im fovealen Bereich 34, welche lediglich etwa 25Hz beträgt, ist bevorzugt vorgesehen, dass die Zeit zwischen zwei unmittelbar aufeinander folgenden Blickfeldbildern etwa 0,04 s beträgt. Je nach Anforderung an die Auflösung können weitere Blickfeldbilder aufgenommen werden, und derart die Zeit zwischen zwei unmittelbar aufeinander folgenden Blickfeldbildern verringert werden, wodurch eine höhere Bewegungsauflösung erreicht wird, und/oder können etwa eine vorgebbare Anzahl an Blickfeldbildern übersprungen werden, oder mit einer geringeren zeitlichen Auflösung aufgenommen werden, wodurch zwar die Bewegungsauflösung sinkt, jedoch auch der rechnerische Aufwand. Durch Vergleich der jeweils unmittelbar nachfolgend Blickfeldbilder kann sowohl eine hohe Bewegungsauflösung, als auch eine geringe Systemkomplexität erreicht werden, da auf Bildauswahlsysteme und Zwischenspeicher verzichtet werden kann.

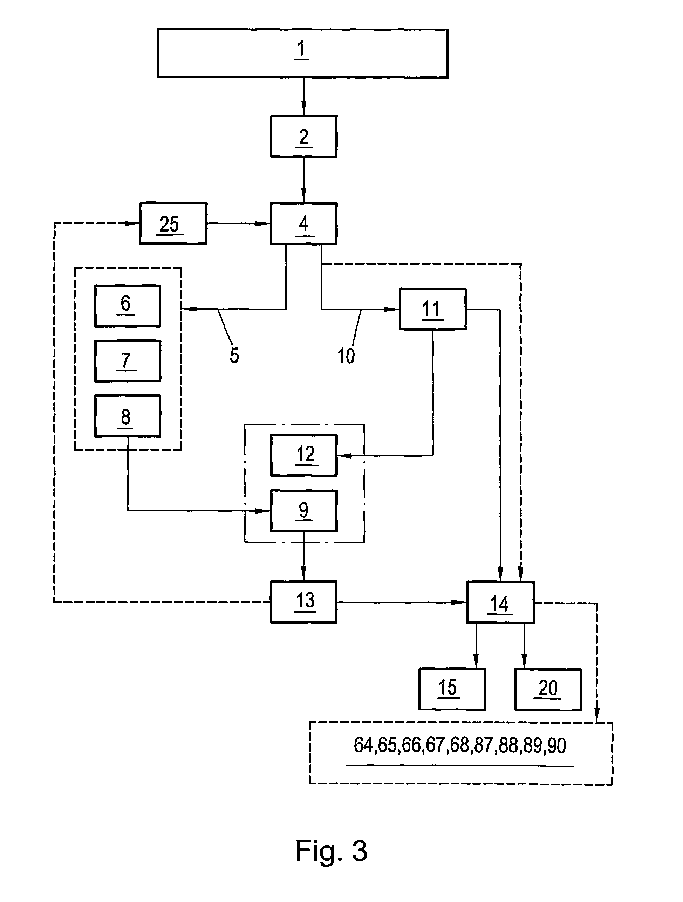

Fig. 3 zeigt ein Blockschaltbild einer besonders bevorzugten Ausführungsform des erfindungsgemäßen Verfahrens, wobei nachfolgend den Verfahrensschritten gemäß dem vorstehend beschriebenen Verfahren gemäß Fig. 1 eine Weiterverarbeitung der ermittelten Daten, daher ob ein Blickpunkt 37, 38 einer Fixation 48, 49 oder einer Sakkade zugeordnet ist vorgesehen ist. Dabei ist vorgesehen, dass der erste Relativabstand 40 zusammen mit den als der ersten Fixation 48 bzw. der ersten Sakkade zugehörig gekennzeichneten Blickpunkten 37, 38 ausgegeben wird. Die Daten werden hiezu für die erste Ausgabe 10 in einem ersten Diagramm 11 und/oder zur zweiten Ausgabe 5 auf einem Blickfeldvideo 9 vorbereitet, wobei bevorzugt vorgesehen ist, dass ein zur Ermittlung der Sichtkoordinaten der Blickpunkte 37, 38 durch das Blickerfassungssystem aufgenommenes Blickfeldvideo 9 ausgegeben wird, und dass die wenigstens der ersten Fixation 48 bzw. wenigstens der ersten Sakkade zugehörigen Blickpunkte 37, 38 in dem Blickfeldvideo 9 dargestellt werden, wodurch eine schnelle und visuelle einfache Bewertung der visuellen Wahrnehmung möglich ist.

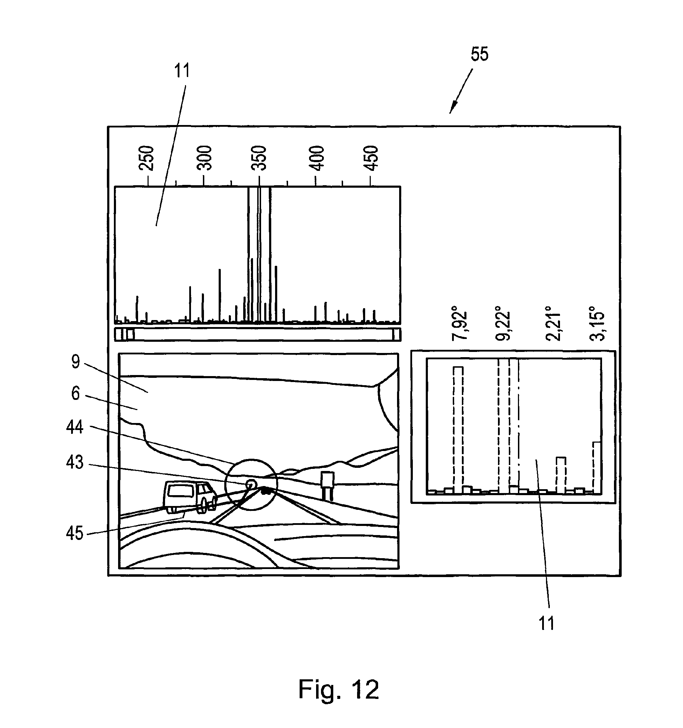

Fig. 12 zeigt einen Screenshot einer bevorzugten Benutzeroberfläche 55 eines Computerprogramms zur Ausführung eines erfindungsgemäßen Verfahrens, wobei links unten das Blickfeldvideo 9 dargestellt wird, in welchem, gemäß nachfolgend beschriebenem Verfahren, blickpunktbezogene Informationen bezüglich der Zugehörigkeit der einzelnen Blickpunkte 37, 38 zu einer Fixation 48, 49 bzw. einer Sakkade ausgegeben werden. Links oberhalb des Blickfeldvideos 9 wird zeitsynchron zu diesem ein erstes Diagramm 11 ausgegeben 12, wobei rechts neben dem Blickfeldvideo 9 - ebenfalls zeitsynchron zum Blickfeldvideo 9 - ein Detailausschnitt des ersten Diagramms 11 ausgegeben wird. Weiters weist die bevorzugte Benutzeroberfläche eine Reihe Steuerungs- und/oder Eingabemittel auf.

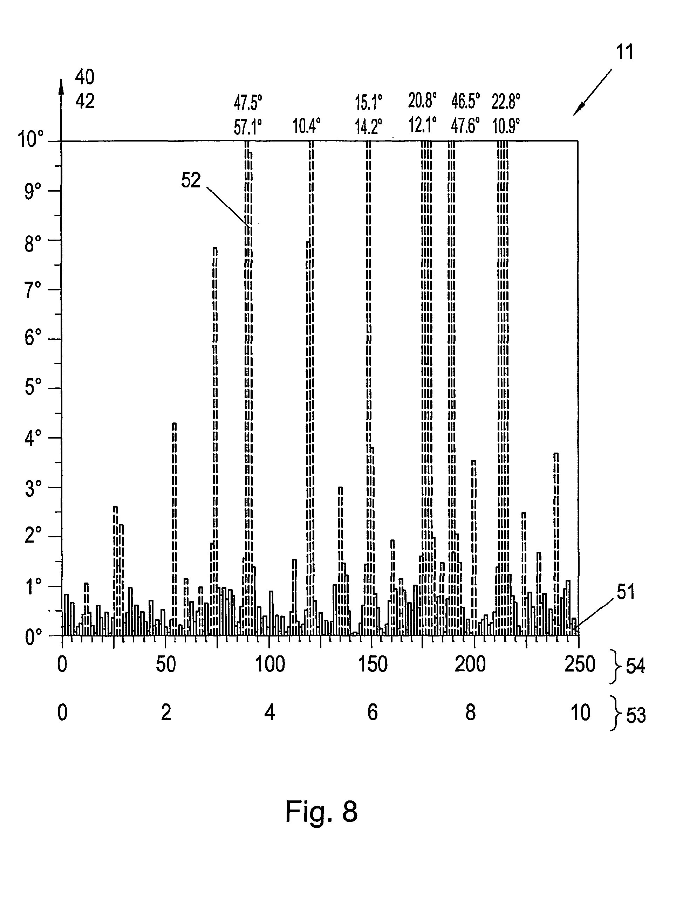

Im ersten Diagramm 11 wird jeweils der erste Relativabstand 40 zwischen zwei, aufeinander folgenden Blickpunkten 37, 38, bzw. zwischen den beiden aufeinanderfolgenden Blickpunkten 37, 38, welche in der Vergleichsvorrichtung auf das Erfüllen des ersten Fixationskriteriums 25 verglichen wurden, über dem zeitlichen Verlauf eines Blickfeldvideos 9 ausgegeben. Fig. 8 zeigt eine bevorzugte Ausführungsform eines ersten Diagramms, wobei auf der Abszisse die Zeit 53 bzw. die fortlaufende Anzahl 54 der Frames, daher der Blickfeldbilder, des Blickfeldvideos 9 aufgetragen ist, und auf der Ordinate der erste Relativabstand 40 bzw. der erste Relativwinkel 42. Die Information ob dieser jeweilige erste Relativabstand 40 zwischen zwei aufeinander folgenden Blickfeldbildern einer Sakkade bzw. einer Fixation 48, 49 zugeordnet wurde, wird weiters durch die farbliche Ausgestaltung bzw. die Helligkeit der einzelnen, dargestellten ersten Relativabstände 40 bzw. der ersten Relativwinkel 42 ausgegeben. Anhand eines derartigen ersten Diagramms 11 können schnell und einfach Blickfeldvideos 9 auf die Wahrnehmung, insbesondere die visuelle Aufmerksamkeit, hin geprüft werden. Es kann weiters vorgesehen sein, dass im ersten Diagramm 11 ein Marker angezeigt wird, um die Stelle zu kennzeichnen, welche derzeit im Blickfeldvideo 9 dargestellt wird, wobei das erste Diagramm 11 ständig mit fortlaufendem Blickfeldvideo 9 neu ermittelt und/oder fortlaufend um den stillstehenden Marker herum als bewegliches und sich veränderndes erstes Diagramm 11 angezeigt wird.

Zusätzlich zur Ausgabe der Daten, ob ein Blickpunkt 37, 38 einer Fixation 48, 49 oder einer Sakkade zugeordnet ist, im ersten Diagramm 11, kann vorgesehen sein die entsprechenden Daten in einem, speziell adaptierten Blickfeldvideo 9 auszugeben, wie dies in Fig. 3 durch die Blöcke 6, 7 und 8 veranschaulicht ist. Bevorzugt sind drei unterschiedliche Ausgabearten vorgesehen, wobei vorgesehen sein kann jeweils lediglich eine dieser drei Ausgabearten auszugeben, es kann aber auch vorgesehen sein, zwei oder alle drei Ausgabearten gleichzeitig darzustellen.

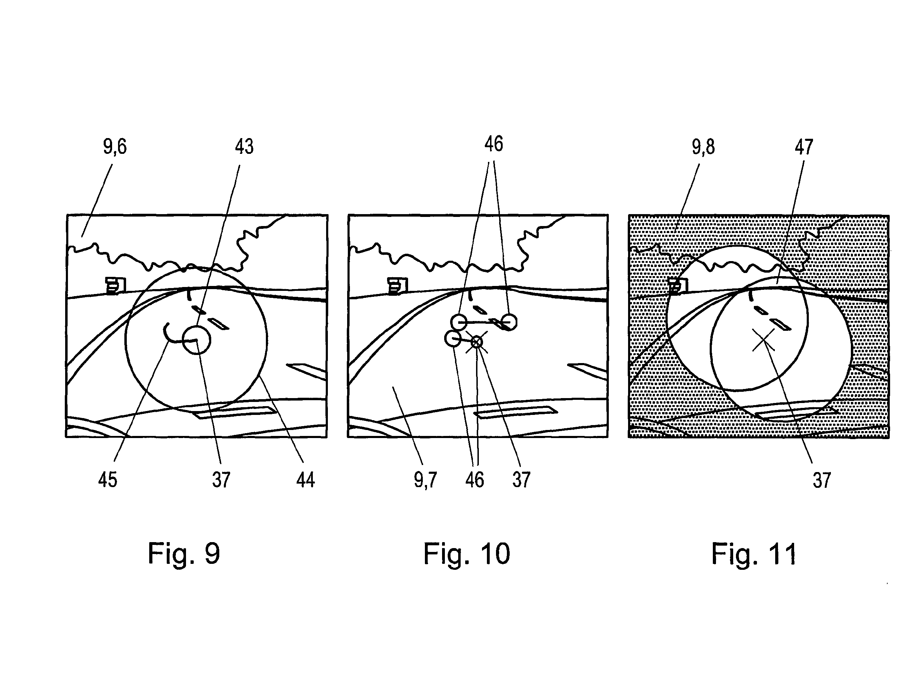

Fig. 9 zeigt eine erste bevorzugte Ausgabeart 6, welche auch in dem im Screenshot gemäß Fig. 12 dargestellt ist, wobei zusammen mit einem jeweils dem aktuell im Blickfeldvideo 9 dargestellten Blickfeldbild korrespondierenden Blickpunkt 37 ein erster Kreis 43 im Wesentlichen gleichmäßig um den Blickpunkt 37 mit dem Radius des ersten Abstands 39 ausgegeben wird, und/oder zusammen mit einem jeweils dem aktuell im Blickfeldvideo 9 dargestellten Blickfeldbild korrespondierenden Blickpunkt 37 ein zweiter Kreis 44 im Wesentlichen gleichmäßig um den Blickpunkt 37 mit dem Radius eines vorgebbaren zweiten Abstands ausgegeben wird, wobei der zweite Abstand vorzugsweise ein zweiter Blickwinkel ist, welcher vorzugsweise einen dem parafovealen Sehen zuzuordnenden Bereich 35 beschreibt, insbesondere mit einem Radius bis 5° und mehr, wodurch bereits bei Betrachtung des Blickfeldvideos 9 die Bereiche erkannt werden können, in denen aufgrund der Sehschärfeverteilung um die zentrale Sehachse geordnete bzw. ungeordnete Wahrnehmung überhaupt möglich ist. Zusätzlich kann vorgesehen sein, dass durch Verbindung aufeinander abfolgender Blickpunkte 37, 38 erste Blickspuren 45 ermittelt werden, welche in dem Blickfeldvideo 9 wenigstens temporär dargestellt werden, daher, dass die Blickspuren 45 nach einer vorgebbaren Zeitdauer wieder aus dem Blickfeldvideo 9, insbesondere kontinuierlich schwächer werdend, ausgeblendet werden, wodurch schnell und einfach erkannt werden kann, welche Bereiche des Blickfeldvideos 9 durch den Betrachter bzw. die Testperson über eine personenabhängige kurze Zeitspanne in Erinnerung bzw. im Kurzzeitgedächtnis sind.

Eine zweite bevorzugte Ausgabeart 7 ist in Fig. 10 dargestellt, wobei vorgesehen ist, die wenigstens der ersten Fixation 48 zugehörig gekennzeichneten Bildpunkte 37 von einem dritten Kreis 46 im Wesentlichen gleichmäßig umgeben darzustellen, wobei der Radius des dritten Kreises 46 eine Funktion der fortlaufenden zeitlichen Dauer der ersten Fixation 48 ist, daher der dritte Kreis wird mit fortlaufender Dauer der jeweiligen Fixation stetig größer. Zusätzlich kann vorgesehen sein, dass die Sakkaden zwischen zwei aufeinander folgenden Fixationen 48, 49 über die Blickpunkte mittels einer Linie verbunden werden. Bevorzugt werden die einzelnen dargestellten Fixationen 48, 49 bzw. Sakkaden nach einer vorgebbaren Zeitdauer wieder aus dem Blickfeldvideo 9 ausgeblendet. Um eine Unterscheidungsmöglichkeit zwischen mehreren gleichzeitig ausgegeben Sakkaden bzw. Fixationen 48, 49 zu gewährleisten kann vorgesehen sein, diese mit unterschiedlichen Farben und/oder Grauwerten zu kennzeichnen, wobei vorgesehen sein kann die Reihenfolge der Fixationen durch unterschiedliche Farben, Grauwerte und/oder Ausbildung der Kreise zu kennzeichnen.

Fig. 11 zeigt eine dritte bevorzugte Ausgabeart 8 der Ausgabe des Blickfeldvideos 9, wobei vorgesehen ist, dieses abgedunkelt darzustellen, und die wenigstens der ersten Fixation 48 zugehörig gekennzeichneten Bildpunkte 37 von einem vierten Kreis 47 im Wesentlichen gleichmäßig umgeben darzustellen, wobei die Fläche des vierten Kreises 47 gegenüber dem angedunkelt dargestellten Blickfeldvideo 9 wenigstens temporär heller dargestellt wird. Dies stellt eine besonders vorteilhafte Ausgestaltung dar, da hiermit - in der Art eines Spotlights bzw. eines Suchscheinwerfers - nur die Bereiche gut sichtbar ausgegeben werden, welche durch den Betrachter auch tatsächlich leidlich wahrgenommen werden bzw. wahrgenommen wurden. Alle anderen Bereiche werden abgedunkelt dargestellt, weil diese auch tatsächlich nicht wahrgenommen wurden.

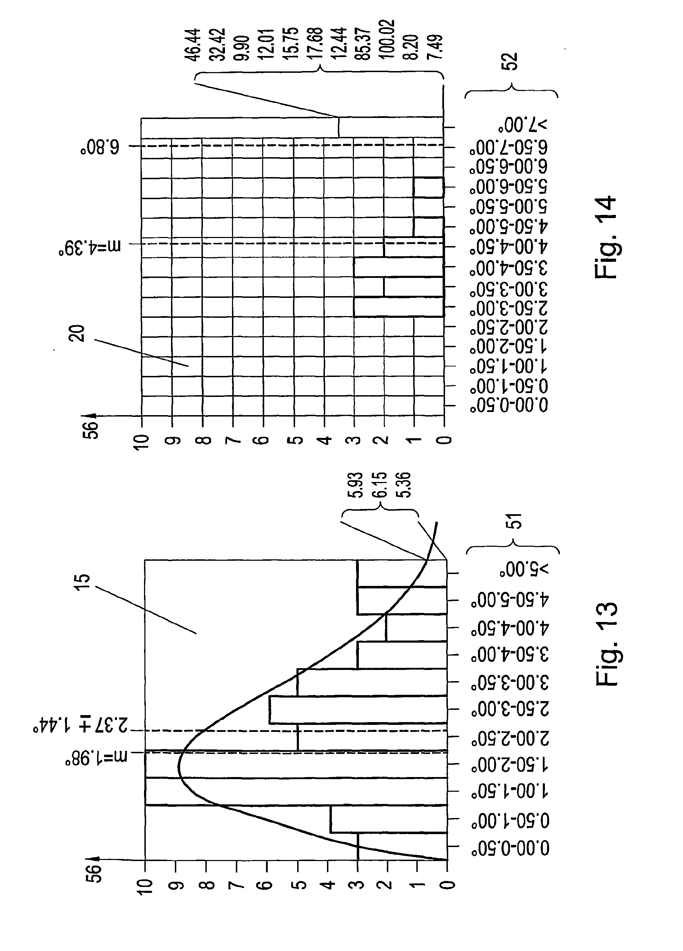

Zusätzlich zur Ausgabe des erfindungsgemäß bearbeiteten Blickfeldvideos 9 bzw. zur Ausgabe 12 des ersten Diagramms 11 (Fig. 8) kann eine Beurteilung einer gesamten Sequenz eines vorgebbaren ersten Abschnitts bzw. des gesamten Blickfeldvideos 9 vorgesehen sein, wobei eine Auswahl 13 (Fig. 3) eines ersten Abschnitts des Blickfeldvideos 9 vorgesehen sein kann. Hiezu werden in einer Auswerteeinheit 14 sämtliche aufeinander folgende Blickpunkte 37, 38, 69, 70, welche jeweils dem ersten Fixationskriterium 25 genügen, zusammen einer ersten Fixation 48 zugeordnet, der Winkelabstand zwischen dem ersten der ersten Fixation 48 zugeordneten Blickpunkt 37 und dem letzten der ersten Fixation 48 zugeordneten Blickpunkt 70 wird ermittelt, und als erster Fixationswinkel 51 ausgegeben (Fig. 13). Zusätzlich wird bevorzugt der Winkelabstand zwischen dem letzten, der ersten Fixation 48 zugeordneten Blickpunkt 70 und einem ersten, einer zweiten Fixation 49 zugeordneten Blickpunkt 73 ermittelt, und als erster Sakkadenwinkel 52 ausgegeben (Fig. 14). Dadurch ist eine gezielte Messung der Aufmerksamkeit für bestimmte vorgebbare Objekte bzw. von Szenen eines Blickfeldvideos 9 möglich, da hiedurch zusätzlich zu dem ersten Messergebnis, daher ob ein Blickpunkt 37 einer Fixation 48 bzw. einer Sakkade zugeordnet ist, auch ein zweites Messergebnis über die zeitliche bzw. lokale Länge der Fixation 48 bzw. der Sakkade ermittelt wird. Bevorzugt ist vorgesehen, dass für einen vorgebbaren ersten Abschnitt des Blickfeldvideos 9 die Häufigkeit der ermittelten Fixationen 48, 49 in Abhängigkeit des Fixationswinkels 51 ausgegeben werden, und/oder dass für den ersten Abschnitt des Blickfeldvideos 9 die Häufigkeit der ermittelten Sakkaden in Abhängigkeit des Sakkadenwinkels 52 bzw. der Zeit ausgegeben werden. Bevorzugt ist dabei vorgesehen, dass die dem ersten Fixationskriterium 25 genügenden bzw. dieses erfüllende Fixationen 48, 49 in einem ersten Fixationsdiagramm 15, sowie bei dem ersten Fixationskriterium 25 ermittelten Sakkaden in einem ersten Sakkadendiagramm 20 ausgegeben werden. Dadurch kann eine gesamte Sequenz bzw. ein vorgebbarer erster Abschnitt einfach und schnell beurteilt werden. Fig. 13 zeigt ein solches erstes Fixationsdiagramm 15, wobei auf der Abszisse der erste Fixationswinkel 51 eingetragen ist, und auf der Ordinate die Häufigkeit 56 in welcher Fixationen 48, 49 mit dem jeweiligen Fixationswinkel 51 auftreten. Das in Fig. 13 dargestellte erste Fixationsdiagramm 15 zeigt dabei die Fixationsschwankungen bei einer Autofahrt. Fig. 14 zeigt ein entsprechendes erstes Sakkadendiagramm 20, wobei auf der Abszisse der erste Sakkadenwinkel 52 eingetragen ist, und auf der Ordinate die Häufigkeit 56 in welcher Sakkaden mit dem jeweiligen Sakkadenwinkel 52 auftreten. Das in Fig. 14 dargestellte erste Sakkadendiagramm 20 zeigt dabei die Sakkadenschwankungen bei einer Autofahrt. Bevorzugt kann vorgesehen sein, dass die Benutzeroberfläche Mittel zur Auswahl eines ersten Abschnitts des Blickfeldvideos 9 aufweist.

Es kann auch vorgesehen sein, dass der erste Abschnitt in Form eines Fensters vorgebbarer Fenstergröße beidseitig des im ersten Diagramm 11 darstellbaren Markers ausgebildet ist, und dass das erste Fixationsdiagramm 15 und/oder das erste Sakkadendiagramm 20 laufend für diesen ersten Abschnitt konstanter Länge aber sich fortlaufend ändernden Inhalts zu errechnen und anzuzeigen.

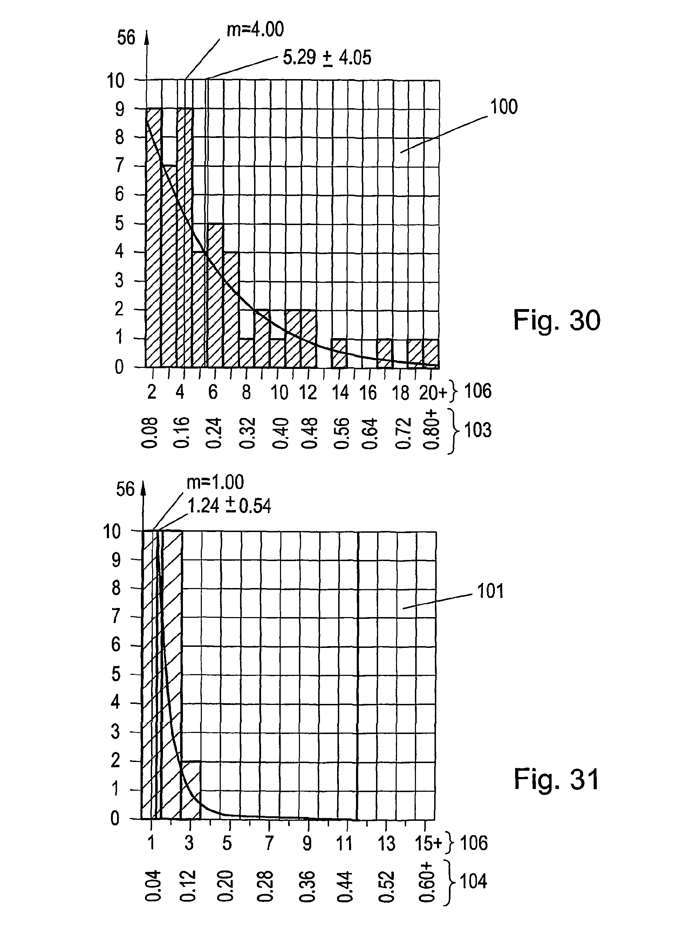

Zusätzlich bzw. alternativ zur Ausgabe des ersten Fixationsdiagramms 15 und/oder des ersten Sakkadendiagramms 20 ist bevorzugt vorgesehen, dass für einen vorgebbaren ersten Abschnitt des Blickfeldvideos 9 sämtliche aufeinander folgende Blickpunkte 37, 38, 69, 70, welche jeweils dem ersten Fixationskriterium 25 genügen, zusammen einer ersten Fixation 48 zugeordnet werden, und dass eine erste Fixationsdauer 103 zwischen dem ersten der ersten Fixation 48 zugeordneten Blickpunkt 37 und dem letzten der ersten Fixation 48 zugeordneten Blickpunkt 70 ermittelt wird, und dass die Häufigkeit 56 der ermittelten Fixationen 48, 49 in Abhängigkeit der ersten Fixationsdauer 103 ausgegeben werden. Fig. 30 zeigt eine besonders bevorzugte Ausgabeart in Form eines Fixationsdauerdiagramms 100, wobei auf der Abszisse die erste Fixationsdauer 103 als zeitliche Dauer einer Fixation 48, 49 aufgetragen ist, wobei als äquivalente Skalierung die Anzahl der Frames 106 bzw. der Bilder eines Blickfeldvideos 9 vorgesehen sein können, und wobei auf der Ordinate die Häufigkeit 56 mit welcher Fixationen 48, 49 mit der jeweiligen Fixationsdauer 103 in dem vorgebbaren ersten Abschnitt des Blickfeldvideos 9 auftreten.

Weiters ist besonders bevorzugt vorgesehen, dass für den ersten Abschnitt des Blickfeldvideos 9 eine erste Sakkadendauer 104 zwischen dem letzten, der ersten Fixation 48 zugeordneten Blickpunkt 70 und einem ersten, einer zweiten Fixation 49 zugeordneten Blickpunkt 73 ermittelt, und dass die Häufigkeit 56 der ermittelten Sakkaden in Abhängigkeit der ersten Sakkadendauer 104 ausgegeben werden. Fig. 31 zeigt eine besonders bevorzugte Ausgabeart in Form eines Sakkadendauerdiagramms 101, wobei auf der Abszisse die erste Sakkadendauer 104 als zeitliche Dauer einer Sakkade aufgetragen ist, wobei als äquivalente Skalierung die Anzahl der Frames 106 bzw. der Bilder eines Blickfeldvideos 9 vorgesehen sein können, und wobei auf der Ordinate die Häufigkeit 56 mit welcher Sakkaden mit der jeweiligen ersten Sakkadendauer 104 in dem vorgebbaren ersten Abschnitt des Blickfeldvideos 9 auftreten. Durch die Ausgabe der Häufigkeit 56 mit der Sakkaden bzw. Fixationen 48, 49 in Abhängigkeit der ersten Sakkadendauer 104 bzw. der ersten Fixationsdauer 103 auftreten kann schnell und einfach analysiert werden welcher Art bzw. Qualität die Blickzuwendungen bei dem ersten Abschnitt sind. So können schnell, einfach und eindeutige objekt- und/oder situationsabhängige Unterschiede erkannt werden.

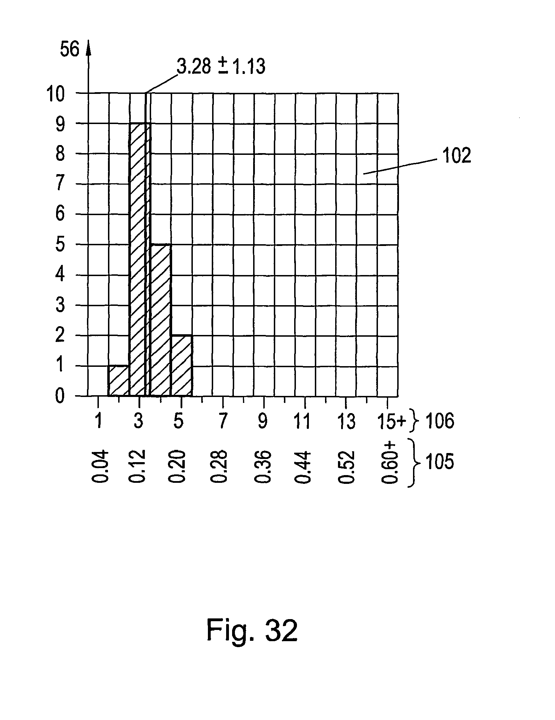

Bei erfindungsgemäßen Verfahren können weiters automatisch Zeitbereiche erkannt weiters, in denen die Augen geschlossen sind. Derartige Zeitbereiche werden durch einen sog. Lidschlag hervorgerufen, bei welchem die Pupille durch das Augenlid temporär verdeckt wird. Es hat sich für die Bewertung sehphysiologischer Zusammenhänge als vorteilhaft herausgestellt, auch die erste Lidschlagdauer 105, als zeitliche Dauer eines Lidschlags, sowie die Häufigkeit mit welcher Lidschläge vorgebbarer erster Lidschlagdauer 105 auftreten zu untersuchen. Fig. 32 zeigt etwa eine bevorzugte Ausgabeart als Lidschlagdiagramm 102, wobei die Häufigkeit 56 mit welcher Lidschläge vorgebbarer erster Lidschlagdauer 105 auftreten, ausgegeben wird. Es hat sich gezeigt, dass aus der geringen Häufigkeit der Lidschläge bzw. der ersten Lidschlagdauer 105 auf einen hohen Grad an Komplexität einer Situation bzw. eines Objekts geschlossen werden kann, und umgekehrt. Zudem kann eine verminderte Lidschlagtätigkeit zu einem Austrocknen der Augen und damit in Verbindung stehenden Augen- und/oder Sehproblemen führen. Daher kann aus dem vorstehend Beschriebenem geschlossen werden, dass ab einer bestimmten Komplexität der Situation eine Verminderung der Sehfähigkeit aufgrund vermindertem Lidschlags zu erwarten ist. Zusätzlich bestehen bei einem Verfahren gemäß Fig. 3 weitere Möglichkeiten der Beurteilung der ermittelten Daten, wie etwa die Ausgabe zufolge weiterer Ausgabemoden 64, 65, 66, 67, 68, 87, 88, 89, 90 welche an weiterer Stelle noch im Detail erläutert werden. Weiters kann auch vorgesehen sein, das erste Fixationskriterium 25 gegen ein zweites Fixationskriterium 26 zu vertauschen und derart wenigstens einen vorgebbaren zweiten Abschnitt des Blickfeldvideos 9 erneut zu untersuchen, wie dies durch die strichlierte Verbindung zwischen der Auswahl 13 des vorgebbaren ersten bzw. zweiten Abschnitts des Blickfeldvideos 9 zum ersten Fixationskriterium 25 veranschaulicht wird.

Wie vorstehend bereits dargelegt, und ausgehend von einer Definition des fovealen Bereichs 34 als den Bereich, in welchem eine geordnete Wahrnehmung möglich ist, ist der erste Blickwinkel 41 des fovealen Bereichs 34 stark Objekt- bzw. Umgebungsabhängig. Beispielsweise werden bekannte Objekte in einer Umgebung, in welcher der Proband mit dem Auftreten dieses Objektes rechnet (etwa einem achteckigen Stoppschild im Straßenverkehr) durch den Probanden sehr schnell wahrgenommen bzw. erkannt. Wohingegen unerwartete bzw. unbekannte Objekte nicht so schnell bzw. eindeutig erkannt bzw. wahrgenommen werden.

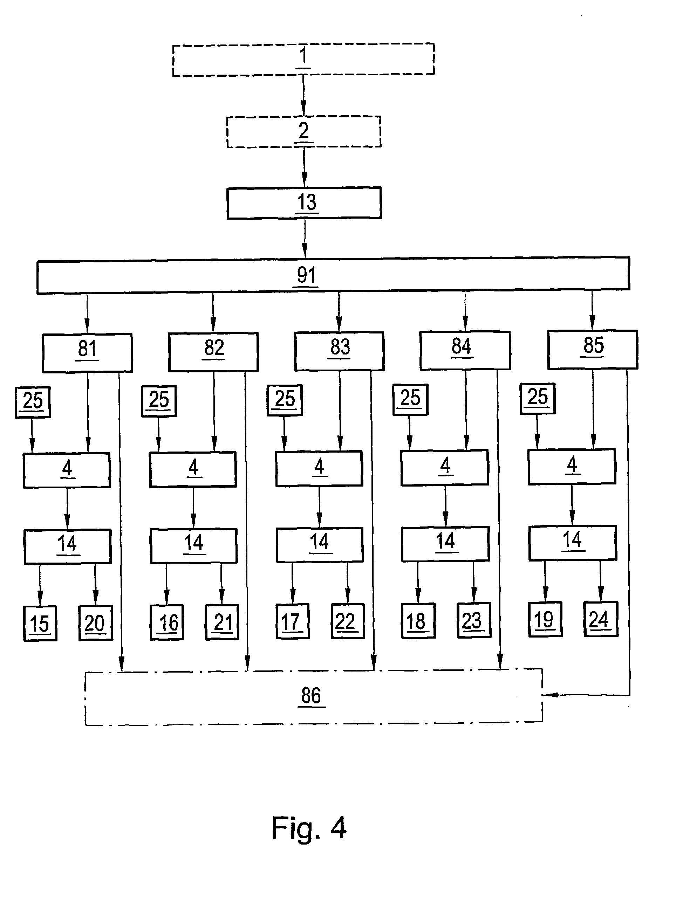

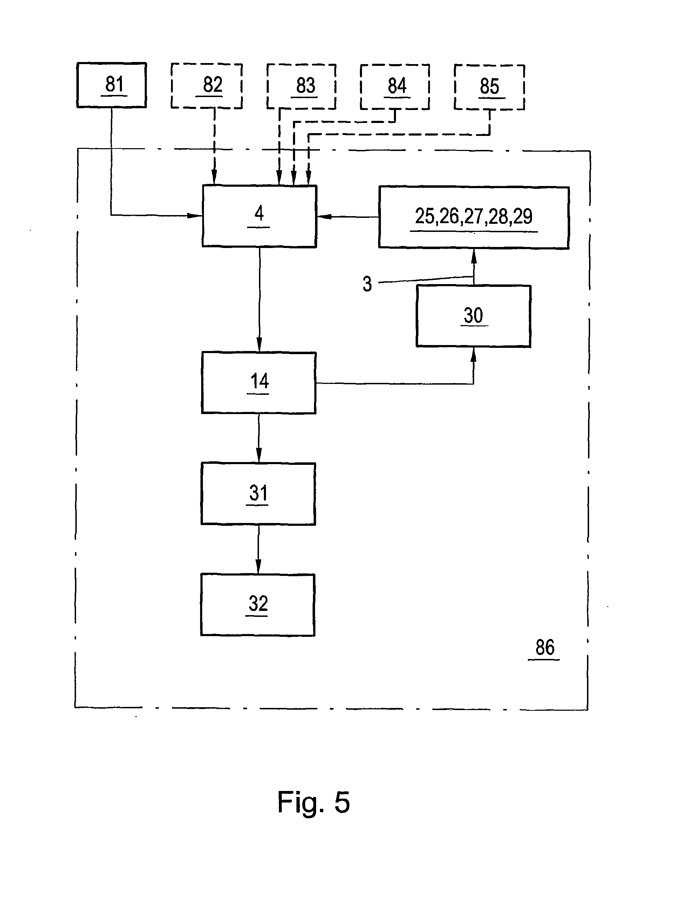

Verfahren zur Messung der Erkennbarkeit vorgebbarer Objekteinheiten, wobei für einen vorgebbaren dritten Abschnitt des Blickfeldvideos 9 sämtliche einer vorgebbaren ersten Objekteinheit zuzuordnende Blickpunkte in einem ersten Objektzwischenspeicher 81 gesammelt werden, und dass das vorstehend beschriebene Verfahren mit den im ersten Objektzwischenspeicher 81 gesammelten Blickpunkten durchgeführt wird. Daher wird für einen vorgebbaren bzw. wählbaren dritten Abschnitt des Blickfeldvideos 9 wenigstens eine Objekteinheit ausgewählt, bevorzugt wird eine vorgebbare Anzahl an Objekteinheiten, beispielsweise wie in Fig. 4 und 5 dargestellt mit fünf Objekteinheiten, ausgewählt. Die Auswahl der Objekteinheiten erfolgt dabei bevorzugt durch einen Benutzer, wobei jedoch auch eine automatische Auswahl der wenigstens einen ersten Objekteinheit vorgesehen sein kann. Bei einer ersten Objekteinheit kann es sich etwa um ein Stoppschild handeln, bei einer zweiten Objekteinheit kann es sich etwa um ein Auto handeln, und bei einer dritten Objekteinheit kann es sich etwa um die Leitlinie einer Strasse handeln. Eine Objekteinheit im Sinne der Erfindung kann auch eine Szene des Blickfeldvideos sein, etwa eine Kurvendurchfahrt.

Fig. 4 zeigt dabei ein Verfahren, bei welchem nach Auswahl 13 eines dritten Abschnitts des Blickfeldvideos 9, dieser dritte Abschnitt des Blickfeldvideos 9 auf Blickpunkte untersucht wird, welche den vorher bestimmten Objekteinheiten zuzuordnen sind bzw. diesen zugeordnet sind. Als einer ersten Objekteinheit zuzuordnende bzw. zugeordnete Blickpunkte werden dabei sämtliche Blickpunkte bezeichnet, welche zwischen dem ersten Blickpunkt einer ersten, die erste Objekteinheit betreffenden, Fixation und dem letzten Blickpunkt einer letzten, die erste Objekteinheit betreffenden, Fixation in dem dritten Abschnitt des Blickfeldvideos auftreten. Nach Auswahl 13 des dritten Abschnitts des Blickfeldvideos 9 wird dieses auf Blickpunkte hin durchsucht (Block 91), welche der ersten Objekteinheit zuzuordnen sind bzw. dieser zugeordnet sind. Dieses Durchsuchen und zuordnen der einzelnen Blickpunkte zu den einzelnen Objekteinheiten kann dabei manuell durch einen Benutzer erfolgen, als auch automatisch mittels eines Computers, beispielsweise mit einer Software zur automatischen Erkennung vorgebbarer optischer Muster, wie etwa Stopschilder, Straßenmarkierungen, Menschen und dergleichen.

Die in den einzelnen Objektzwischenspeichern 81, 82, 83, 84, 85 abgelegten Blickpunkte werden dann, wie in Fig. 4 dargestellt, jeweils zufolge einem vorstehend beschriebenen Verfahren verarbeitet und bewertet. Nach der Auswertung wird jeweils ein Fixationsdiagramm 15, 16, 17, 18, 19 und ein Sakkadendiagramm 20, 21, 22, 23, 24 je Objektzwischenspeicher ausgegeben. Daher bei dem bevorzugten Verfahren gemäß Fig. 4 wird bevorzugte ein erstes Fixationsdiagramm 15, ein zweites Fixationsdiagramm 16, ein drittes Fixationsdiagramm 17, ein viertes Fixationsdiagramm 18 und ein fünftes Fixationsdiagramm 19 ausgegeben, sowie ein erstes Sakkadendiagramm 20, ein zweites Sakkadendiagramm 21, ein drittes Sakkadendiagramm 22, ein viertes Sakkadendiagramm 23 und ein fünftes Sakkadendiagramm 24. Es ist dadurch möglich unterschiedliche Objekte hinsichtlich deren Wahrnehmungsqualität zu unterscheiden bzw. zu bewerten. Insbesondere ist es dadurch möglich unterschiedlichen Objekten eine sog. Aufforderungscharakteristik zuzuordnen, daher wie stark wird meine Aufmerksamkeit durch das betreffenden Objekt angezogen. Daher gibt es Objekte, welche durch deren Gestaltung die Aufmerksamkeit des Betrachters auf sich ziehen bzw. bündeln, wohingegen andere Objekte die Aufmerksamkeit des Betrachters unberührt lassen. Die Erkenntnis wie ein Objekt beschaffen sein muss um Aufmerksamkeit zu erregen, bzw. welche Objekte Aufmerksamkeit durch einen Betrachter anziehen ist in vielen Bereich des täglichen Lebens wichtig, etwa bei der Gestaltung von Fußgängerübergängen, bei der Gestaltung von Sicherheitsbekleidung, bei der Straßenführung, oder etwa bei der Gestaltung von Werbemitteln. Zusätzlich bestehen bei einem Verfahren gemäß Fig. 4 weitere Möglichkeiten der Beurteilung der ermittelten Daten, wie etwa die Ausgabe zufolge weiterer Ausgabemoden 64, 65, 66, 67, 68, 87, 88, 89, 90 welche an weiterer Stelle noch im Detail erläutert werden.

Bekannte bzw. erwartete Objekte werden bereits bei weitaus größerem erstem Blickwinkel 41 voll als solches Objekt erkannt, als etwa bis jetzt noch unbekannte bzw. unerwartete Objekte. Daher kann die Sehschärfe und damit auch der foveale Bereich 34 für ein erstes Objekt bzw. eine erste Umgebung größer oder kleiner sein als für ein zweites Objekt bzw. eine zweite Umgebung. Die Größe der für das spezifische Objekt notwendigen Sehschärfe daher einen sehr aussagekräftigen Wert für die Wahrnehmbarkeit eines Objekts bzw. einer szenischen Abfolge dar, wobei sich der Begriff der szenischen Abfolge auf sämtliche zeitlichen Abfolgen beziehen kann, etwa das Durchfahren eines Straßenbereichs, oder das Betrachten einer Werbeanzeige.

Je größer der Bereich um die zentrale Sehachse in bzw. bei welcher ein Objekt erkannt wird, umso schneller und leichter wird dieses durch einen Betrachter wahrgenommen, daher umso höher ist auch die Wahrscheinlichkeit, dass dieses durch den Betrachter auch tatsächlich als solches Objekt richtig wahrgenommen wird, auch wenn für benachbarte Objekte etwa das erste Fixationskriterium 25 nicht erfüllt ist. Beispielsweise könnte ein Betrachter bei einem flüchtig streifenden Blick über ein Gebäude die am Dach angebrachte Reklame für eine bekannte Softdrinkfirma oder eine bekannte Fastfoodkette einwandfrei als solche erkennen, während die Form des Daches nicht wahrgenommen wird.

Eine Ansführungsform der Erfindung betrifft daher weiters ein Verfahren zur Wahrnehmungsmessung von vorgebbaren Objekteinheiten, wobei das vorstehend beschriebe Verfahren weiters für wenigstens einen vorgebbaren zweiten Abschnitt des Blickfeldvideos 9 mit wenigstens einem vorgebbaren, vom ersten Fixationskriterium 25 unterschiedlichen, zweiten Fixationskriterium 26 durchgeführt wird, wodurch die Qualität vorgebbarer Objekte und/oder von Blicksequenzen hinsichtlich deren Wahrnehmbarkeit durch einen Betrachter ermittelt werden kann. Fig. 5 zeigt eine bevorzugte Ausführungsform eines derartigen Verfahrens als Blockschaltbild, wobei die einzelnen Verfahrensschritte als gemeinsamer strichpunktiert dargestellter Block 86 zusammengefasst dargestellt sind. Bei einer bevorzugten Ausführungsform der Erfindung ist vorgesehen, dass der zweite Bereich ident ist mit dem dritten Bereich, wobei besonders bevorzugt vorgesehen ist, dass entsprechende im Block 86 zusammengefasste Verfahren auf die im ersten Objektzwischenspeicher 81 abgelegten bzw. gesammelten, einer vorgebbaren ersten Objekteinheit zuzuordnenden bzw. zugeordneten Blickpunkte anzuwenden, wie dies auch in Fig. 5 dargestellt ist.

Bei der Ausführungsform gemäß Fig. 5 ist vorgesehen, dass der zweite Abschnitt des Blickfeldvideos 9, bzw. der Inhalt des ersten Objektzwischenspeichers 81, des zweiten Objektzwischenspeichers 82, des dritten Objektzwischenspeichers 83, des vierten Objektzwischenspeichers 84 und/oder des fünften Objektzwischenspeichers 85, seriell nacheinander in der Vergleichsvorrichtung 4 und der Auswertung 14 mit jeweils unterschiedlichen Fixationskriterien 25, 26, 27, 28, 29, daher nacheinander wenigstens jeweils mit einem ersten Fixationskriterium 25, einem zweiten Fixationskriterium 26, einem dritten Fixationskriterium 27, einem vierten Fixationskriterium 28 und einem fünften Fixationskriterium 29, in Form einer Verfahrensschleife 30 zur Variation des Fixationskriteriums verarbeitet wird, wobei die Ergebnisse in einem ersten Speicher 31 abgelegt und dann ausgegeben werden.

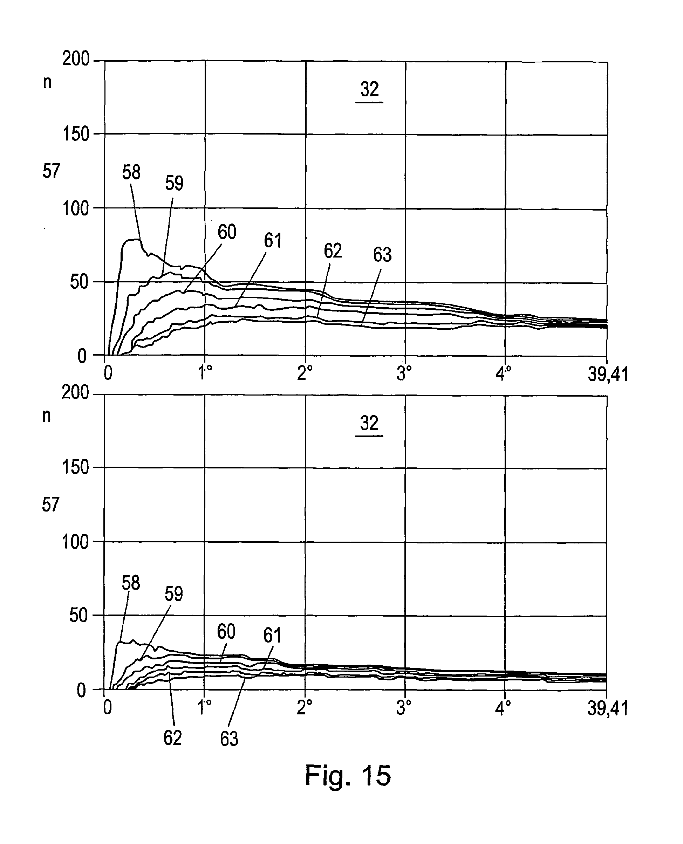

Bevorzugt ist dabei vorgesehen, die ermittelten Daten bez. die vom jeweiligen Fixationskriterium 25, 26, 27, 28, 29 abhängige Objektwahrnehmung auszugeben. Vorzugsweise ist dabei vorgesehen, dass die Häufigkeit der Fixationen 48, 49 in Abhängigkeit wenigstens des ersten und des zweiten Fixationskriteriums 25, 26 als erste Kurve 58 mit konstanter erster Zeitdauer und als zweite Kurve 59 mit konstanter zweiter Zeitdauer ausgegeben wird. Fig. 15 zeigt ein solches zweites Diagramm, welches auch als sog. Fixations-Level-Diagramm 32 bezeichnet wird, bei welchem auf der Abszisse der erste Abstand 39 bzw. der erste Blickwinkel 41, und auf der Ordinate die Anzahl 57 an Fixationen aufgetragen ist, und wobei jede der dargestellten sechs Kurven 58, 59, 60, 61, 62, 63 jeweils mit unterschiedlicher erste Zeitdauer ermittelt wurden, daher bei der ersten Kurve 58 beträgt der Abstand zwischen dem ersten Blickfeldbild 37 und dem zweiten Blickfeldbild 38 einen Frame bzw. ein Blickfeldbild, daher ist das zweite Blickfeldbild 38 das auf das erste Blickfeldbild 37 unmittelbar folgende Blickfeldbild. Bei der zweiten Kurve 59 beträgt der Abstand zwischen dem ersten Blickfeldbild 37 und dem zweiten Blickfeldbild 38 zwei Frames. Bei der dritten Kurve 60 beträgt der Abstand zwischen dem ersten Blickfeldbild 37 und dem zweiten Blickfeldbild 38 drei Frames. Bei der vierten Kurve 61 beträgt der Abstand zwischen dem ersten Blickfeldbild 37 und dem zweiten Blickfeldbild 38 vier Frames. Bei der fünften Kurve 62 beträgt der Abstand zwischen dem ersten Blickfeldbild 37 und dem zweiten Blickfeldbild 38 fünf Frames. Bei der sechsten Kurve 63 beträgt der Abstand zwischen dem ersten Blickfeldbild 38 und dem zweiten Blickfeldbild 38 sechs Frames. Fig. 15 zeigt dabei zwei unterschiedliche Fixations-Level-Diagramme 32, welche jeweils unterschiedliche Szenen oder Objekte betreffen. Aus derartigen Fixations-Level-Diagrammen 32 können schnell die wahrnehmungsspezifischen Unterschiede unterschiedlicher Objekte in Abhängigkeit des ersten Abstandes 39 bzw. des ersten Blickwinkels 41, sowie der ersten Zeitdauer ermittelt werden, wodurch eine wissenschaftliche Bewertung bzw. Messung der unterschiedlichen Wahrnehmbarkeit von Objekten ermöglicht wird. Es ist dadurch möglich unterschiedlichen Objekten eine sog. Aufforderungscharakteristik zuordnen, daher wie stark wird meine Aufmerksamkeit durch das betreffende Objekt angezogen.

Zur weiteren Bewertung und Analyse des Blickverhaltens und der Objektwahrnehmung können, wie vorstehend bereits dargelegt, noch weitere Ausgabeformate vorgesehen sein, wie diese in den Fig. 16 bis 20, sowie 24 bis 27 veranschaulicht sind.

In Fig. 16 werden, vorzugsweise für eine erste Objekteinheit, sämtliche Blickpunkte dargestellt, daher sämtlich im ersten Objektzwischenspeicher abgelegten Blickpunkte werden ohne besondere Bewertung und/oder Gewichtung dargestellt. Durch eine derartige, auch als "Dots" 64 bezeichnete, Darstellung ist es für einen geübten Betrachter bereits möglich eine Reihe von Aussagen über die Qualität des betrachteten Objekts zu tätigen. Die grau hinterlegte Fläche, kann - sowohl bei dieser Darstellungsart, als auch bei sämtlichen weiteren Darstellungsarten gemäß den Fig. 16 bis 20, sowie 24 bis 27 - zur besseren Verständlichkeit auch mit einem Bild des ersten Objekts hinterlegt werden, wobei berücksichtigt werden muss, dass bei dynamischer Annäherung dann die dargestellten Blickpunkte nicht tatsächlich auf die in dem hinterlegten Bild dargestellten Bereichen gerichtet sein müssen.

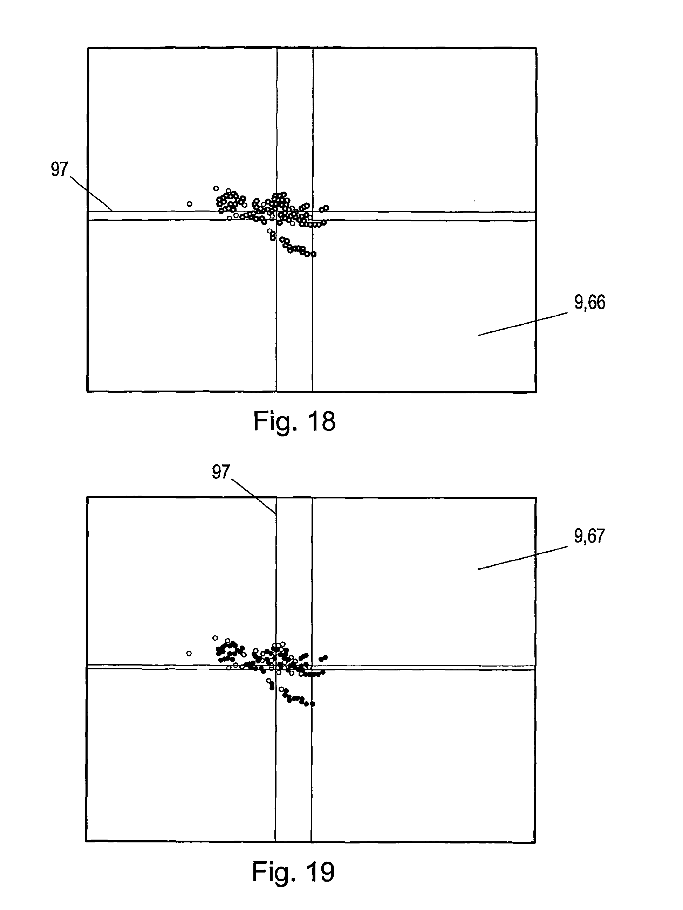

In Fig. 18 werden, vorzugsweise für eine erste Objekteinheit, sämtliche Blickpunkte dargestellt, daher sämtlich im ersten Objektzwischenspeicher abgelegten Blickpunkte werden dargestellt, wobei sämtliche einer Fixation zugeordneten Blickpunkte gekennzeichnet dargestellt, wobei bevorzugt vorgesehen ist, dass diese gegenüber der Umgebung in einem gut wahrnehmbaren Kontrast und/oder in einem gut wahrnehmbaren Helligkeitsunterschied und/oder in einer von der Umgebung abweichenden Farbe dargestellt werden. Die derart gekennzeichnet dargestellten bzw. ausgegebenen Blickpunkte werden auch als Fixdots 66 bezeichnet. Dadurch ist eine besonders feine und differenzierte Beurteilung der Wahrnehmungsqualitäten eines ersten Objekts möglich. In Fig. 18 ist darüber hinaus ein erstes Achsenkreuz 97 dargestellt, welches den Mittelpunkt und/oder den Schwerpunkt der Blickpunkte kennzeichnet.

In Fig. 19 werden ebenfalls sämtliche im ersten Objektspeicher abgelegte Blickpunkte dargestellt, wobei sämtliche einer Fixation vorgebbarer Länge zugeordnete Blickpunkte gekennzeichnet dargestellt, wobei bevorzugt vorgesehen ist, dass diese gegenüber der Umgebung in einem gut wahrnehmbaren Kontrast und/oder in einem gut wahrnehmbaren Helligkeitsunterschied und/oder in einer von der Umgebung abweichenden Farbe dargestellt werden. Die derart gekennzeichnet dargestellten bzw. ausgegebenen Blickpunkte werden auch als "weighted Dots" 67 bezeichnet. Durch Veränderung der vorgebbaren Länge der Fixation kann schnell und einfach analysiert werden, wie sich die Wahrnehmungsqualitäten eines ersten Objekts in Abhängigkeit der Länge der einzelnen Fixationen ändert. In Fig. 19 ist darüber hinaus weiters auch das erste Achsenkreuz 97 dargestellt.

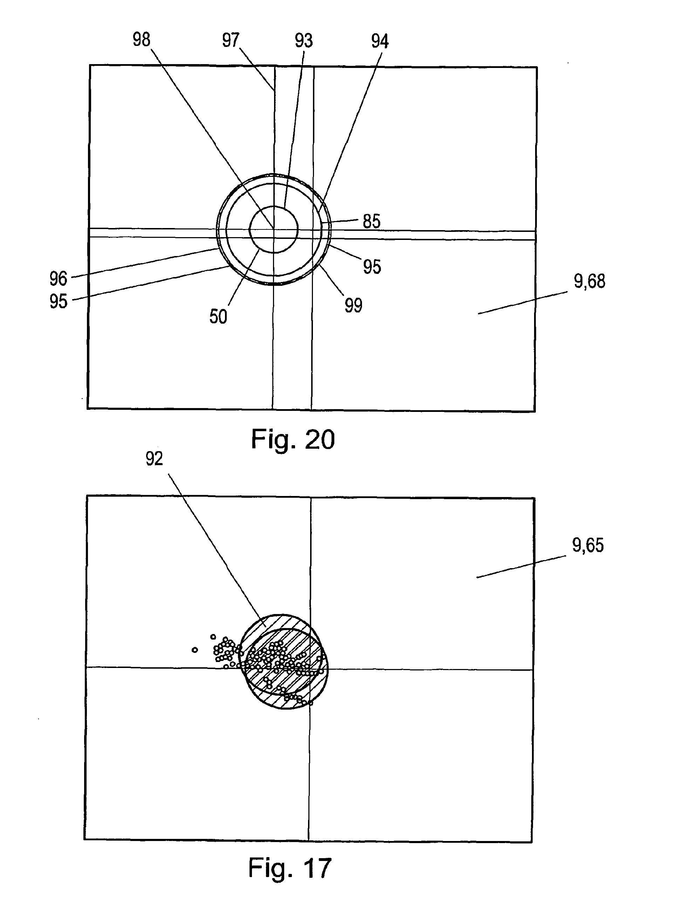

Fig. 20 zeigt eine bevorzugte Ausgabeform, welche zusätzlich zu den weiters beschriebenen Ausgabeformen angewandt werden kann. Dabei sind um den Mittelpunkt 98 der Fixationspunkte des ersten Achsenkreuzes 97 eine vorgebbare Anzahl Kreise eingezeichnet. Bevorzugt, und wie dargestellt, ist ein siebenter Kreis 93 dargestellt, dessen Durchmesser derart ausgebildet ist, dass der siebente Kreis 93 fünfzig Prozent der Blickpunkte umfasst. Weiters ist ein achter Kreis 94 dargestellt, dessen Durchmesser derart ausgebildet ist, dass der siebente Kreis 93 fünfundachtzig Prozent der Blickpunkte umfasst. Der weiters dargestellte neune Kreis 95 ist derart ausgebildet, dass dieser fünfundneunzig Prozent der Blickpunkte umfasst, und der zehnte Kreis 96 ist derart ausgebildet, dass dieser neunundneunzig Prozent der Blickpunkte umfasst. Diese - auch als "zone-angle" 68 bezeichnete - Darstellung kann mit jeder der anderen Ausgabeformen kombiniert werden, und ermöglicht eine schnelle objektspezifische Bewertung der Wahrnehmungsqualität.

In Fig. 17 werden äquivalent zu Fig. 16 die im ersten Objektzwischenspeicher abgelegten Blickpunkte dargestellt, wobei weiters die Bereiche, welche einer Fixation nach einer dieser vorausgegangenen "langen" Sakkade zugeordnet werden, durch einen sechsten Kreis 92 dargestellt werden, dessen Fläche gegenüber der Umgebung in einem gut wahrnehmbaren Kontrast und/oder in einem gut wahrnehmbaren Helligkeitsunterschied und/oder in einer von der Umgebung abweichenden Farbe dargestellt werden. Der Mittelpunkt des sechsten Kreises 92 ist durch den Schwerpunkt der der jeweils betreffenden Fixation zugeordneten Blickpunkte gegeben. Eine derartige Darstellungsform wird auch als "Fixationsdominanz" 65 bezeichnet. Die Länge einer langen Sakkade ist über einen vorgebbaren ersten Sakkadenwinkel 52 gegeben. Alternativ kann auch eine Sakkadenzeitspanne vorgegeben werden, ab deren Überschreiten eine Sakkade eine lange Sakkade ist. Der Durchmesser des sechsten Kreises ist vorgebbar und liegt bevorzugt in einem Bereich, welcher vorzugsweise einen dem parafovealen Sehen zuzuordnenden Bereich 35 beschreibt. Durch diese Darstellung kann schnell und auch für einen ungeübten Betrachter eindringlich ausgegeben werden auf welchen Bereichen eines Objekts besonderer Augenmerk des Betrachters ruht. Es kann auch vorgesehen sein, dass der sechste Kreis 92 nur dargestellt wird, wenn die für das Erkennen eines Objekts durch den Betrachter erforderlichen Merkmale hinsichtlich Fixation 48, 49 und Fixationsdauer 103 erfüllt sind. Dadurch wird schnell und eindeutig erkennbar ob ein Objekt durch den Betrachter lediglich beiläufig gesehen, wahrgenommen oder tatsächlich erkannt wird bzw. wurde.

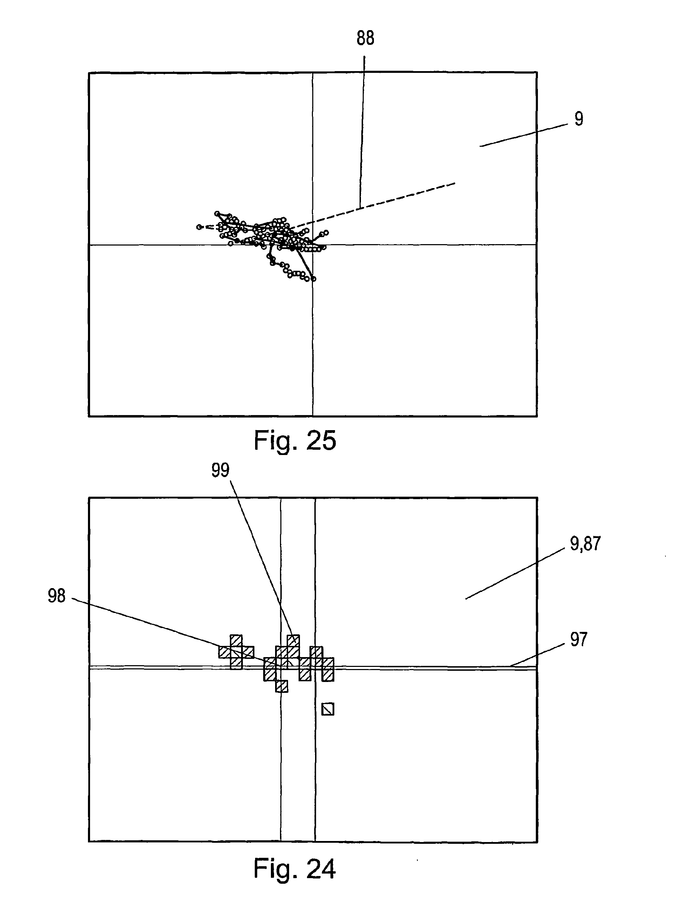

In Fig. 25 zeigt eine zehnte bevorzugte Ausgabe 88, wobei ausschließlich die Sakkaden zwischen einzelnen Fixation dargestellt werden, indem der jeweils letzte Blickpunkt einer ersten Fixation mit dem jeweils ersten Blickpunkt einer zweiten Fixation mit einer Linie verbunden dargestellt wird, wobei die Länge der Sakkade farblich unterschiedlich dargestellt werden kann, wodurch ein Betrachter die Bereiche mit langen Wahrnehmungsdefiziten schnell erkennen kann.

In Fig. 24 zeigt eine neunte bevorzugte Ausgabe 87, wobei das Blickfeld mit einem Raster vorgebbarer Größe und/oder Ausgestaltung überzogen wird, und die einzelnen Rastersegmente 99 werden bezüglich der Häufigkeit der in dieses auftretenden Blickpunkte durch eine vorgebbare Ausgestaltung hinsichtlich deren Helligkeit, deren Farbe und/oder deren Strukturierung gekennzeichnet.

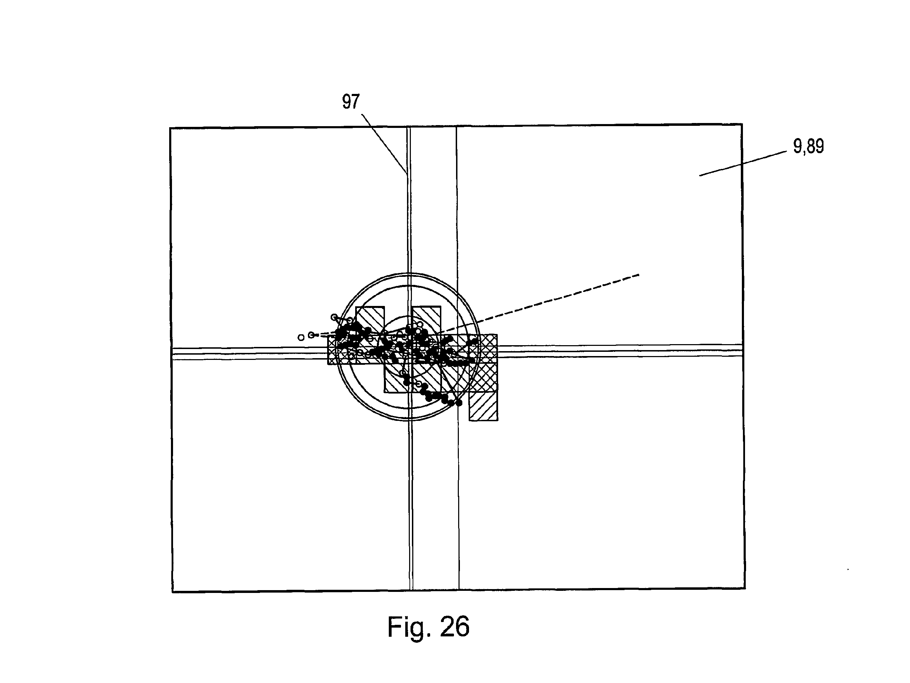

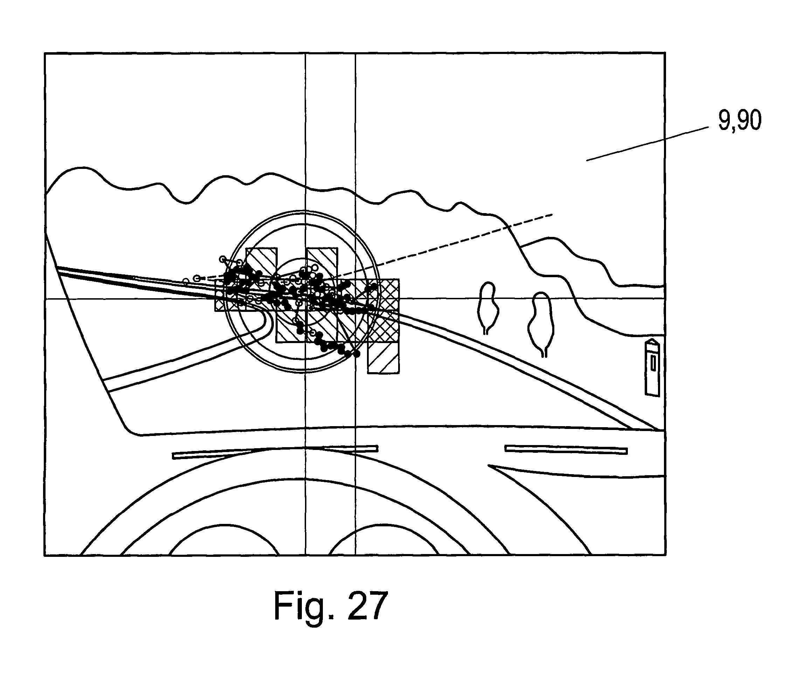

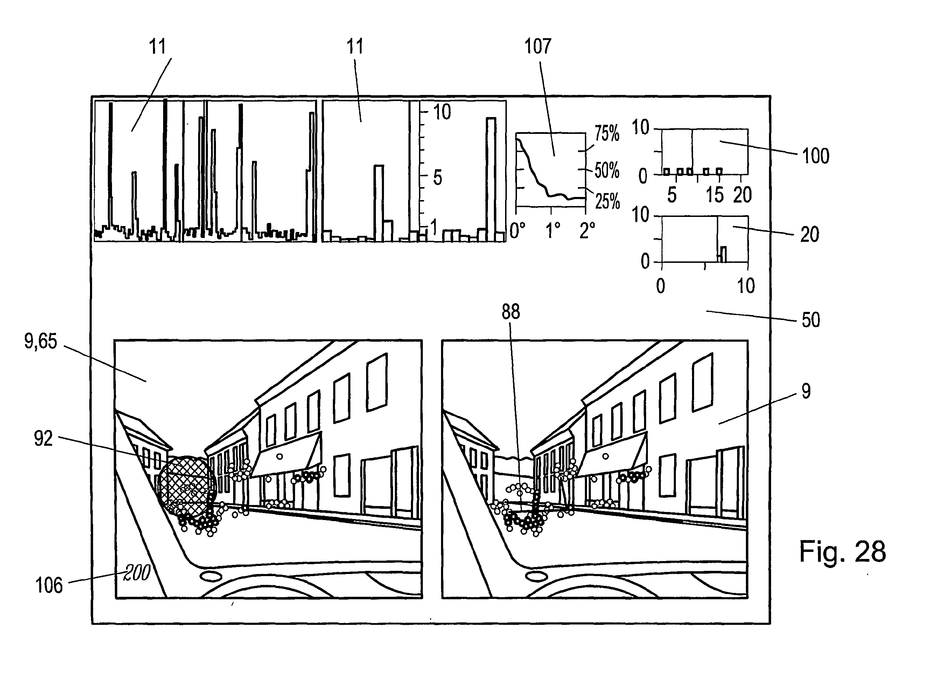

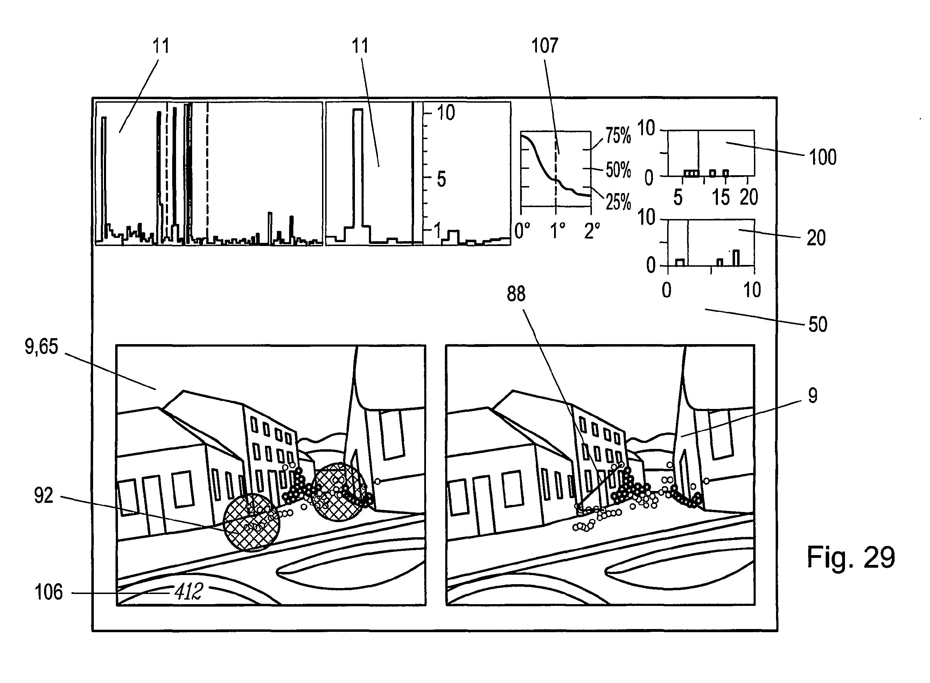

In Fig. 26 zeigt eine elfte bevorzugte Ausgabe, wobei die Ausgabemethoden gemäß den Fig. 17, 19, 20, 24 und 25 überlagert dargestellt werden, wodurch besonders viele Informationen auf nur einem Bild dargestellt werden, und der Betrachter ein Objekt bzw. eine Einzelszene besonders schnell und einfach beurteilen kann. Fig. 27 zeigt eine zwölfte bevorzugte Ausgabe, wobei dieselbe Ansicht wie Fig. 26 die Darstellung zur besseren Verständlichkeit mit einem Bild des ersten Objekts - in diesem Fall einer ersten Szene - hinterlegt ist. Zusätzlich zu den bereits beschriebenen Auswerte- und/oder Ausgabeverfahren ist besonders bevorzugt ein weiteres im folgenden beschriebenes Auswerte- und/oder Ausgabeverfahren vorgesehen, welches im besonderen Maße zur Auffindung der Komplexität einer szenischen Abfolge geeignet ist. Dabei handelt es sich - wie anhand zweier Beispiele in den Fig. 28 und 29 erläutert werden wird - um eine besonders bevorzugte Kombination bereits beschriebener Auswerte- und/oder Ausgabeverfahren, welche bevorzugt um weitere vorteilhafte Auswertungen und/oder Ausgaben erweitert sind.

Die Fig. 28 und 29 zeigen eine bevorzugte Ausgabemaske 50, wobei Steuerungstools und weitere Ausgabefelder ausgeblendet wurden, und lediglich textlich beschrieben werden. Zu erkennen sind die beiden neben einander dargestellten Blickfeldvideos 9, wobei eines der beiden Blickfeldvideos 9 eine Darstellung gemäß Fig. 17 und das andere Blickfeldvideo 9 eine Darstellung gemäß Fig. 25 zeigt. Weiters ist ein erstes Diagramm 11 vorgesehen, sowie eine Detaildarstellung eines solchen ersten Diagramms 11. Die Ausgabemaske 50 umfasst weiters ein erstes Sakkadendiagramm 20 und ein erstes Fixationsdauerdiagramm 100 für die untersuchte Zeit, vorzugsweise 2 s, jedoch vorgebbar. Weiters ist ein zweites Diagramm 107 vorgesehen, in welchem ausgegeben wird, wie viele Blickpunkte - daher die Häufigkeit der Blickpunkte - in welchem ersten Relativabstand 40 um eine zentrale Sehachse angeordnet sind. Weiters ist bevorzugt vorgesehen, jeweils für eine aktuell dargestellte Blickfeldvideosequenz die erste Fixationsdauer 103, den Sakkadenwinkel 52, sowie einen Wert für die Komplexität der Sequenzanzugeben, wobei der Wert für die Komplexität als Summe der in einer vorgebbaren Zeitspanne, in der Regel eine Sekunde, gemessenen ersten Relativwinkel 42 ermittelt und ausgegeben wird. Dadurch ist schnell und einfach möglich zu bestimmen ob ein Proband mit einer Situation überfordert ist, oder nicht. Sobald der Wert für die Komplexität einen vorgebbaren Grenzwert überschreitet, kann davon ausgegangen werden, dass keine geordnete Wahrnehmung der Objekte mehr stattfindet. Im Straßenverkehr kann eine derartige Situation zu verheerenden Folgen führen. Mittels eines derartigen vorstehend beschriebenen Verfahrens und einer derartigen Auswertung kann nicht nur eine Situation bewertet werden, sondern auch einfach und schnell ermittelt werden, ob ein Proband etwa fahrtüchtig ist.

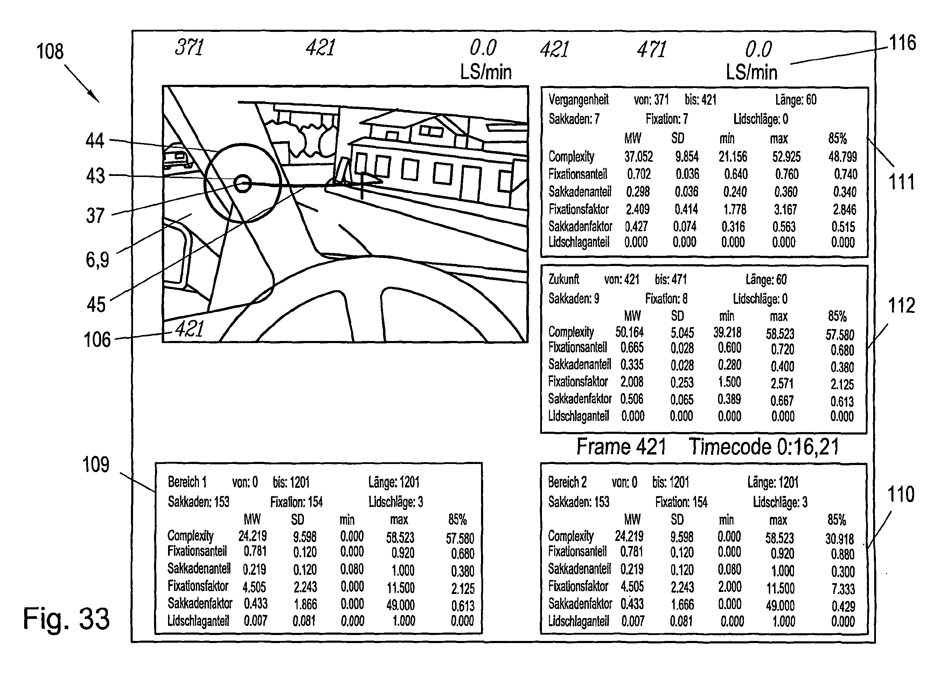

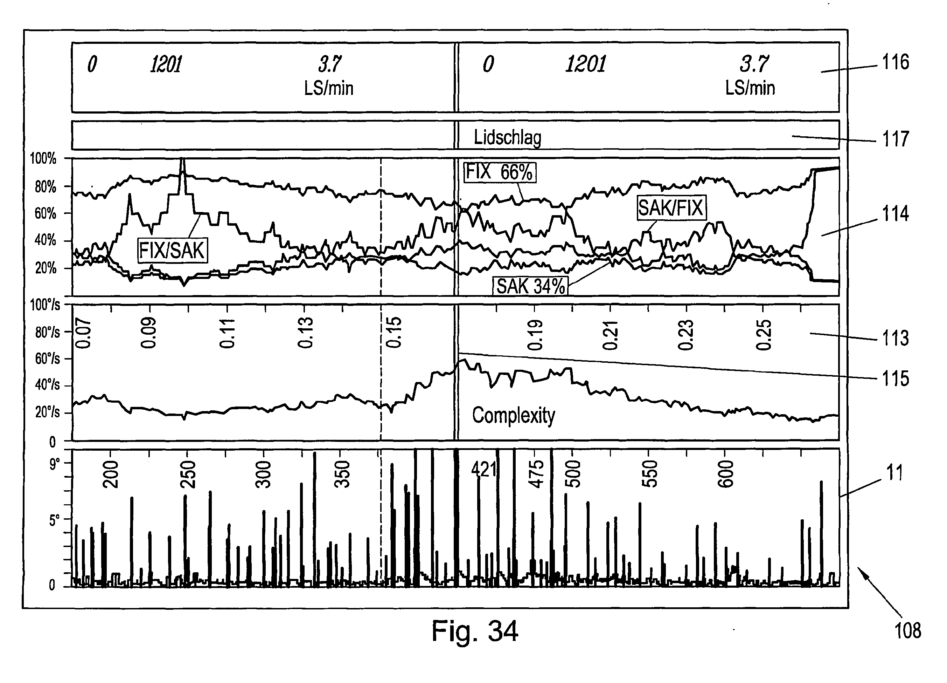

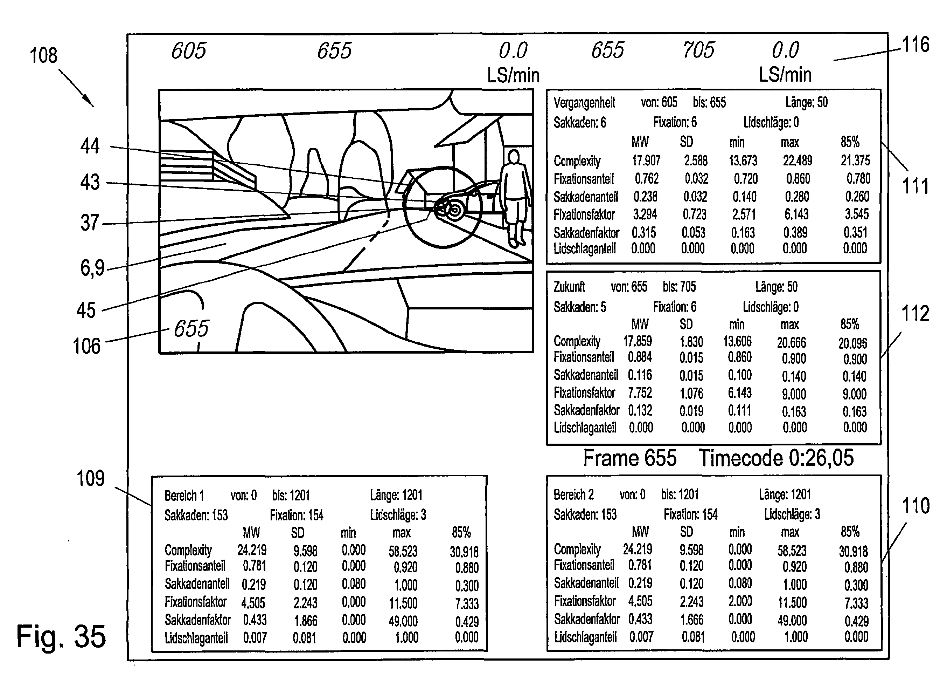

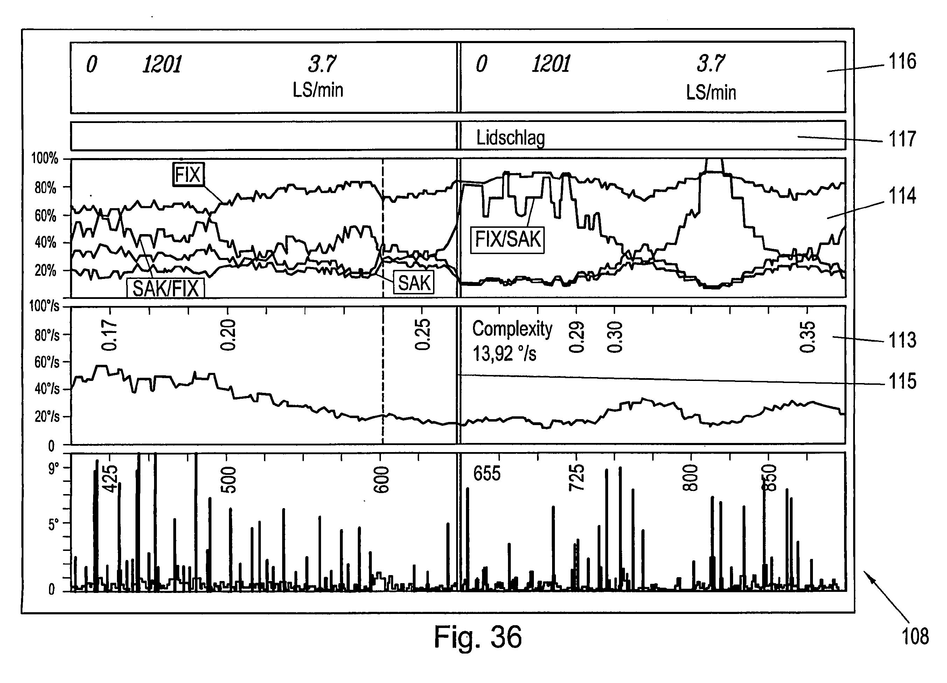

Die Fig. 33 bis Fig. 36 zeigen Beispiele einer bevorzugten Ausgabemaske 108 eines bevorzugten Analysewerkzeugs, wobei die Fig. 33 und 34 eine Einheit bilden bzw. einander zugeordnet sind, und die Fig. 35 und 36 ebenfalls eine Einheit bilden bzw. einander zugeordnet sind. Die Fig. 33 und 35 zeigen jeweils ein Blickfeldvideo 9, welches zufolge der ersten bevorzugten Ausgabeart 6 eines Blickfeldvideos 9 mit einem ersten und einem zweiten Kreis 43, 44, gemäß Fig. 9 dargestellt ist, wobei auch die weiteren beschriebenen bevorzugten Ausgaben eines Blickfeldvideos 9 vorgesehen sein können. Im Blickfeldvideo 9 wird dabei die Nummer des augenblicklich dargestellten Blickfeldbilds bzw. Frames als fortlaufende Nummer 106 dargestellt, wodurch eine exakte Zuordnung des jeweils dargestellten Blickfeldbilds innerhalb des Blickfeldvideos 9 möglich ist. Weiters werden statistische Daten zu dem aktuellen Blickfeldvideo 9 ermittelt, vorzugsweise von einem Computer errechnet, und in einem ersten Bereichsstatistikblock 109 und zweiten Bereichsstatistikblock 110, sowie in einem Vergangenheitsstatistikblock 111 und einem Zukunftsstatistikblock 112 dargestellt. In dem ersten und zweiten Bereichsstatistikblock 109, 110 sind dabei die statistischen Daten für jeweils einen frei wählbaren temporären Bereich des Blickfeldvideos 9 dargestellt. In dem Vergangenheitsstatistikblock 111 sind die statistischen Daten für einen vorgebbaren Zeitbereich vor dem im jeweiligen Moment dargestellten Blickfeldbild dargestellt, und im Zukunftsstatistikblock 112 die statistischen Daten für einen vorgebbaren Zeitbereich nachfolgend dem im jeweiligen Moment dargestellten Blickfeldbild.

In den einzelnen Statistikblöcken 109, 110, 111, 112, daher dem ersten und zweiten Bereichsstatistikblock 109, 110, dem Vergangenheitsstatistikblock 111 und dem Zukunftsstatistikblock 112, werden eine Komplexität (Complexity), ein Fixationsanteil, ein Sakkadenanteil, ein Fixationsfaktor, ein Sakkadenfaktor und ein Lidschlaganteil, wobei MD den arthmetischen Mittelwert, SD die Standardabweichung bzw. Varianz, min das Minimum, max das Maximum und 85% das 85-te Perzentil der jeweiligen Werte für den in dem jeweiligen Statistikblock 109, 110, 111, 112 ausgewählten Zeitbereich des Blickfeldvideos 9 bezeichnen.

Die Komplexität bezeichnet hiebei die Summe aller Blickbewegungen in dem ausgewählten Zeitbereich des Blickfeldvideos 9, vorzugsweise in Grad/Zeiteinheit, daher etwa °/s. Der Fixationsanteil bezeichnet den zeitlichen Anteil des ausgewählten Zeitbereichs des Blickfeldvideos 9, welcher Fixationen zugeordnet werden kann, in Verhältnis zur gesamten zeitlichen Dauer des ausgewählten Zeitbereichs des Blickfeldvideos 9; und der Sakkadenanteil bezeichnet den zeitlichen Anteil des ausgewählten Zeitbereichs des Blickfeldvideos 9, welcher Sakkaden zugeordnet werden kann, in Verhältnis zur gesamten zeitlichen Dauer des ausgewählten Zeitbereichs des Blickfeldvideos 9. Der Fixationsanteil und der Sakkadenanteil können jeweils Werte zwischen Null und Eins annehmen und ergeben in deren Summe Eins, da jeweils nur Bereiche zu deren Ermittlung herangezogen werden, welche keine Lidschläge, daher vollständige temporäre Abdunkelungen des Auges, aufweisen.

Der Fixationsfaktor ist das Verhältnis des Anteils an Fixationen zu dem Anteil an Sakkaden zu jedem Zeitpunkt, und der Sakkadenfaktor ist das Verhältnis des Anteils an Sakkaden zu dem Anteil an Fixationen zu jedem Zeitpunkt. Der Lidschlaganteil ist der zeitliche Anteil der Lidschläge an dem ausgewählten Zeitbereich.

Die Anzahl der Fixationen, Sakkaden und Lidschläge werden in den jeweiligen Statistikblöcken 109, 110, 111, 112 vorzugsweise und wie dargestellt zusätzlich als diskreter Wert dargestellt.

In Fig. 34 sind die statistischen Daten zu dem Blickfeldvideo 9 und den Statistikblöcken 109, 110, 111, 112 gemäß Fig. 33 in graphischer Form dargestellt, und in Fig. 36 sind die statistischen Daten zu dem Blickfeldvideo 9 und den Statistikblöcken 109, 110, 111, 112 gemäß Fig. 35 in graphischer Form dargestellt. Bevorzugt und wie dargestellt ist dabei ein erster Diagramm 11 zur graphischen Darstellung der Fixationen und Sakkaden vorgesehen. Weiters wird der jeweiligen Wert der Komplexität in einem Komplexitätsdiagramm 113 dargestellt. Die weiteren Werte des Fixationsanteils, Sakkadenanteils, Fixationsfaktors und/oder Sakkadenfaktors sind weiters in einem Übersichtsdiagramm 114 dargestellt. Durch den zentral angeordneten Doppelbalken 115 wird die im jeweils korrespondierenden Blickfeldvideo 9 dargestellte Stelle gekennzeichnet. Weiters wird der Lidschlag sowohl als numerischer Lidschlagwert 116, als auch in Form eines Lidschlagbalkens 117 dargestellt. Das Analysewerkzeug gemäß den Fig. 33 bis 36 ist besonders bevorzugt zur Findung und detaillierten Untersuchung von Stellen mit Informationsverlusten bedingt durch hohe Komplexität oder häufigen fovealen, zentralen Blickbindungen vorgesehen. Durch die Ausgabe besonders aussagekräftiger statistischer Werte und deren unmittelbare Zuordenbarkeit zu dem dargestellten Blickfeldvideo 9 sind qualitative Tiefenanalysen der realen Informationsaufnahme und verfeinerte Betrachtungen von unterschiedlichen Informationsausfällen und/oder Informationsdefekten möglich. Dadurch kann der Grad der visuellen Wahrnehmung ermittelt werden, wodurch weitere medizinische und neurophysiologische Untersuchungen ermöglicht werden.

In einer Weiterführung der Erfindung kann vorgesehen sein, visuelle Wahrnehmungsgrößen mit individuellen Stressparametern (humanphysiologische Daten) und physikalischen Bewegungs- und Zustandgrößen zusammen auszuwerten, wodurch die vorstehend beschriebenen Verfahren in einem noch weiteren Umfeld der Stress- und Verhaltensforschung eingesetzt werden können.

Eine Ansführungsform der Erfindung betrifft weiters ein Verfahren zur Überwachung der visuellen Wahrnehmung wenigstens eines ersten, vorzugsweise humanoiden, Benutzers, wobei durch wenigstens eine erste Umgebungskamera wenigstens ein erstes Umgebungsvideo der Umgebung des ersten Benutzers aufgenommen wird, dass das erste Umgebungsvideo auf das Vorhandensein wenigstens eines vorgebbaren ersten Musters, vorzugsweise Verkehrszeichen, untersucht wird, dass mit einem Verfahren nach einem der Ansprüche 1 bis 20 ermittelt wird, ob das erste Fixationskriterium 25 bei mit dem ersten Muster wenigstens bereichsweise deckungsgleichen Blickpunkten erfüllt wird, und dass bei einem Nichterfüllen des ersten Fixationskriteriums bei dem ersten Muster wenigstens eine erste Steuerungs- und/oder Regelungsschaltung aktiviert wird. Dadurch kann eine Maschine den Sichtbereich eines Benutzers zusammen mit dem Blickverhalten des Benutzers überwachen, und etwa feststellen, ob gewisse vorgebbare Bereiche bzw. Muster durch den Benutzer wahrgenommen werden bzw. wurde. Etwa kann ein Auto den Straßenbereich auf Verkehrszeichen absuchen, und kontrollieren ob der Fahrer die Verkehrszeichen tatsächlich wahrgenommen hat. Ist dies nicht der Fall, kann das Auto etwa mittels eines Signallichts oder einem Signalton darauf aufmerksam machen, bzw. das Auto selbsttätig anhalten, wenn etwa ein Stoppschild übersehen wurde.

Für die Implementierung eines derartigen Verfahrens ist es notwendig, dass die Pupillenbewegung des Benutzers bzw. des Fahrers aufgenommen wird, weshalb ein entsprechendes Blickerfassungssystem vorgesehen ist. Auch wenn mit Blickerfassungssystemen, welche starr mit dem Kopf verbunden sind, die besten Ergebnisse erzielt werden, kann etwa auch ein Blickerfassungssystem vorgesehen sein, welches die Pupillenbewegung bzw. die Blickrichtung durch eine Vielzahl von um den Benutzer herum angeordneter Kameras, aufnimmt. Bevorzugt vorgesehen ist daher die Anwendung in Bereichen, in denen der Benutzer ohnedies eine Schutzbrille bzw. einen Helm trägt, da ein solches Blickerfassungssystem dann einfach in den Helm bzw. die Schutzbrille integriert werden kann. Mögliche Einsatzbereiche sind etwa, sich schnell bewegende Maschinen, wie etwa Drehbänke, oder Seilereimaschinen, Helme für Kampfflugzeugpiloten, bei welchen das Flugzeug selbst die Umgebung auf Zielen und Gefahren absucht, und den Piloten nur dann darauf aufmerksam macht, wenn er diese nicht wahrgenommen hat. Derartige Systeme könnten auch in die Helme von Rennfahrern integriert werden, und etwa auf die Mustererkennung von Flaggensignalen durch Streckenposten und anderes optimiert werden.

### Bezugszeichen:

1. Ermitteln der Blickpunkte bzw. der Sichtkoordinaten
2. Einlesen der Sichtkoordinaten
3. Einlesen des Fixationskriteriums
4. Ermitteln ob Fixation oder Sakkade
5. zweite Ausgabe
6. erste bevorzugte Ausgabeart
7. zweite bevorzugte Ausgabeart
8. dritte bevorzugte Ausgabeart
9. Blickfeldvideo
10. erste Ausgabe
11. erstes Diagramm
12. Ausgabe des ersten Diagramms
13. Auswahl eines ersten Abschnitts des Blickfeldvideos
14. Auswerteeinheit
15. erstes Fixationsdiagramm
16. zweites Fixationsdiagramm
17. drittes Fixationsdiagramm
18. viertes Fixationsdiagramm
19. fünftes Fixationsdiagramm
20. erstes Sakkadendiagramm
21. zweites Sakkadendiagramm
22. drittes Sakkadendiagramm
23. viertes Sakkadendiagramm
24. fünftes Sakkadendiagramm
25. erstes Fixationskriterium
26. zweites Fixationskriterium
27. drittes Fixationskriterium
28. viertes Fixationskriterium
29. fünftes Fixationskriterium
30. Verfahrensschleife zur Variation des Fixationskriteriums
31. erster Speicher
32. Fixations-Level-Diagramm
33. Auge
34. fovealer Bereich
35. parafovealer Bereich
36. peripherer Bereich
37. erster Blickpunkt
38. zweiter Blickpunkt
39. erster Abstand
40. erster Relativabstand
41. erster Blickwinkel
42. erster Relativwinkel
43. erster Kreis
44. zweiter Kreis
45. erste Blickspuren
46. dritter Kreis
47. vierter Kreis
48. erste Fixation
49. zweite Fixation
50. Ausgabemaske
51. erster Fixationswinkel
52. erster Sakkadenwinkel
53. Zeit
54. fortlaufende Anzahl der Frames des Blickfeldvideos
55. Benutzeroberfläche
56. Häufigkeit
57. Anzahl der Fixationen
58. erste Kurve
59. zweite Kurve
60. dritte Kurve
61. vierte Kurve
62. fünfte Kurve
63. sechste Kurve
64. Dots
65. Fixationsdominanz
66. Fixdots
67. weighted dots
68. zone-angle
69. dritter Blickpunkt erste Fixation
70. letzter Blickpunkt erste Fixation
71. Blickpunkt, zwischen erster und zweiter Fixation
72. Blickpunkt, zwischen erster und zweite Fixation
73. Blickpunkt, erster Blickpunkt zweite Fixation
74. Blickpunkt, zweiter Blickpunkt zweite Fix
75. Blickpunkt, dritter Blickpunkt zweite Fix
76. erste Kamera
77. zweite Kamera
78. Augenbild
79. Blickfeldbild
80. Kopf der Testperson
81. erster Objektzwischenspeicher
82. zweiter Objektzwischenspeicher
83. dritter Objektzwischenspeicher
84. vierter Objektzwischenspeicher
85. fünfter Objektzwischenspeicher
86. Block
87. neunte bevorzugte Ausgabe
88. zehnte bevorzugte Ausgabe
89. elfte bevorzugte Ausgabe
90. zwölfte bevorzugte Ausgabe
91. durchsuchen des Blickfeldvideos auf Blickpunkte, Block Fig. 4
92. sechser Kreis
93. siebenter Kreis
94. achter Kreis
95. neunter Kreis
96. zehnter Kreis
97. erstes Achsenkreuz
98. Mittelpunkt der Fixationspunkte
99. Rastersegmente
100. erstes Fixationsdauerdiagramm
101. eines Sakkadendauerdiagramms
102. Lidschlagdiagramm
103. erste Fixationsdauer
104. erste Sakkadendauer
105. erste Lidschlagdauer
106. Anzahl der Frames
107. zweites Diagramm
108. Ausgabemaske
109. erster Bereichsstatistikblock
110. zweiter Bereichsstatistikblock
111. Vergangenheitsstatistikblock
112. Zukunftsstatistikblock
113. Komplexitätsdiagramm
114. Übersichtsdiagramm
115. Doppelbalken
116. Lidschlagwert
117. Lidschlagbalken

## Patentansprüche

1. Verfahren zur Wahrnehmungsmessung, insbesondere zur Messung der individuellen visuellen Aufmerksamkeit, wobei - insbesondere durch ein Blickerfassungssystem ermittelte - wenigstens erste, einem ersten Blickfeldbild eines Blickfoldvideos zugeordnete, Sichtkoordinaten eines ersten Blickpunktes (37) und wenigstens zweite, einem zweiten Blickfeldbild des Blickfeldvideos zugeordnete, Sichtkoordinaten eines zweiten Blickpunktes (38) verarbeitet werden, wobei das zweite Blickfeldbild nachfolgend dem ersten Blickfeldbild aufgenommen wurde, wobei die zweiten Sichtkoordinaten des zweiten Blickpunktes (38) mit den ersten Sichtkoordinaten des ersten Blickpunktes (37) in einer Vergleichsvorrichtung auf die Erfüllung wenigstens eines ersten vorgebbaren Fixationskriteriums (25) geprüft werden, wobei die ersten und die zweiten Blickpunkte (37, 38) bei Erfüllung des ersten Fixationskriteriums (25) einer der geordneten Wahrnehmung zuzuordnenden ersten Fixation (48) zugeordnet und gekennzeichnet werden, wobei die ersten und die zweiten Blickpunkte (37, 38) bei Nichterfüllung des ersten Fixationskriteriums (25) einer der ungeordneten Wahrnehmung zuzuordnenden ersten Sakkade zugeordnet und gekennzeichnet werden, wobei das zur Ermittlung der Sichtkoordinaten der Blickpunkte (37, 38) durch das Blickerfassungssystem aufgenommenes Blickfeldvideo (9) ausgegeben wird, wobei die wenigstens der ersten Fixation (48) bzw. wenigstens der ersten Sakkade zugehörigen Blickpunkte (37, 38) in dem Blickfeldvideo (9) dargestellt werden.

2. Verfahren nach Anspruch 1, **dadurch gekennzeichnet, dass** der erste und der zweite Blickpunkt (37, 38) als der ersten Fixation (48) bzw. der ersten Sakkade zugehörig gekennzeichnet ausgegeben werden.

3. Verfahren nach Anspruch 1 oder 2, **dadurch gekennzeichnet, dass** das erste Fixationskriterium (25) vorgebbar ist, insbesondere dass das erste Fixationskriterium (25) ein vorgebbarer erster Abstand (39) um den ersten Blickpunkt (37) ist, und ss der erste Relativabstand (40) zwischen dem ersten Blickpunkt (37) und dem zweiten Blickpunkt (38) ermittelt wird, und dass wenn der erste Relativabstand (40) geringer als der erste Abstand (39) ist, der erste und der zweite Blickpunkt (37, 38) der ersten Fixation (48) zugeordnet werden, wobei der erste Abstand (39) insbesondere ein erster Blickwinkel (41) ist, welcher vorzugsweise einen dem fovealen Sehen zugeordneten Bereich (34) beschreibt, insbesondere zwischen 0,5° und 1,5°, vorzugsweise etwa 1°, und der Abstand zwischen dem ersten Blickpunkt (37) und dem zweiten Blickpunkt (38) insbesondere ein erster Relativwinkel (42) ist.

4. Verfahren nach einem der Ansprüche 1 bis 3, **dadurch gekennzeichnet, dass** das zweite Blickfeldbild nach einer vorgebbaren ersten Zeitdauer, insbesondere zwischen 0,005s und 0,1s, vorzugsweise zwischen 0,02s und 0,04s, nachfolgend, insbesondere unmittelbar nachfolgend, dem ersten Blickfeldbild aufgenommen wurde.

5. Verfahren nach Anspruch 3 oder 4, **dadurch gekennzeichnet, dass** der erste Relativabstand (40) zusammen mit den als der ersten Fixation (48) bzw. der ersten Sakkade zugehörig gekennzeichneten Blickpunkten (37, 38) ausgegeben wird, vorzugsweise dass der erste Relativabstand (40) in einem ersten Diagramm (11) über dem zeitlichen Verlauf eines Blickfeldvideos (9) ausgegeben wird.

6. Verfahren nach einem der Ansprüche 1 bis 5, **dadurch gekennzeichnet, dass** zusammen mit einem jeweils dem aktuell im Blickfeldvideo (9) dargestellten Blickfeldbild korrespondierenden Blickpunkt (37) ein erster Kreis (43) im Wesentlichen gleichmäßig um den Blickpunkt (37) mit dem Radius des ersten Abstands (39) ausgegeben wird, insbesondere dass zusammen mit einem jeweils dem aktuell im Blickfeldvideo (9) dargestellten Blickfeldbild korrespondierenden Blickpunkt (37) ein zweiter Kreis (44) im Wesentlichen gleichmäßig um den Blickpunkt (37) mit dem Radius eines vorgebbaren zweiten Abstands ausgegeben wird, welcher zweite Abstand vorzugsweise ein zweiter Blickwinkel ist, welcher insbesondere einen dem parafovealen Sehen zuzuordnenden Bereich (35) beschreibt, vorzugsweise 3° bis 5°.

7. Verfahren nach einem der Ansprüche 1 bis 6, **dadurch gekennzeichnet, dass** durch Verbindung aufeinander abfolgender Blickpunkte (37, 38) erste Blickspuren (45) ermittelt werden, welche in dem Blickfeldvideo (9) wenigstens temporär dargestellt werden.

8. Verfahren nach einem der Ansprüche 1 bis 7, **dadurch gekennzeichnet, dass** die wenigstens der ersten Fixation (48) zugehörig gekennzeichneten Bildpunkte (37) von einem dritten Kreis (46) im Wesentlichen gleichmäßig umgeben dargestellt werden, und dass der Radius des dritten Kreises (46) eine Funktion der fortlaufenden zeitlichen Dauer der ersten Fixation (48) ist.

9. Verfahren nach einem der Ansprüche 1 bis 8, **dadurch gekennzeichnet, dass** das Blickfeldvideo (9) abgedunkelt dargestellt wird, dass die wenigstens der ersten Fixation (48) zugehörig gekennzeichneten Bildpunkte (37) von einem vierten Kreis (47) im Wesentlichen gleichmäßig umgeben dargestellt werden, und dass die Fläche des vierten Kreises (47) gegenüber dem angedunkelt dargestellten Blickfeldvideo (9) wenigstens temporär heller dargestellt wird.

10. Verfahren nach einem der Ansprüche 1 bis 9, **dadurch gekennzeichnet, dass** für einen vorgebbaren ersten Abschnitt des Blickfeldvideos (9) sämtliche aufeinander folgende Blickpunkte (37, 38, 69, 70), welche jeweils dem ersten Fixationskriterium (25) genügen, zusammen einer ersten Fixation (48) zugeordnet werden, und dass der Winkelabstand zwischen dem ersten der ersten Fixation (48) zugeordneten Blickpunkt (37) und dem letzten der ersten Fixation (48) zugeordneten Blickpunkt (70) ermittelt wird, und als erster Fixationswinkel (51) ausgegeben wird, und dass für den ersten Abschnitt des Blickfeldvideos (9) vorzugsweise die Häufigkeit (56) der ermittelten Fixationen (48, 49) in Abhängigkeit des Fixationswinkels ausgegeben werden.

11. Verfahren nach Anspruch 10, **dadurch gekennzeichnet, dass** für den ersten Abschnitt des Blickfeldvideos (9) der Winkelabstand zwischen dem letzten, der ersten Fixation (48) zugeordneten Blickpunkt (70) und einem ersten, einer zweiten Fixation (49) zugeordneten Blickpunkt (73) ermittelt, und als erster Sakkadenwinkel (52) ausgegeben wird, und dass für den ersten Abschnitt des Blickfeldvideos (9) vorzugsweise die Häufigkeit (56) der ermittelten Sakkaden in Abhängigkeit des Sakkadenwinkels ausgegeben werden.

12. Verfahren nach einem der Ansprüche 1 bis 11, **dadurch gekennzeichnet, dass** für einen vorgebbaren ersten Abschnitt des Blickfeldvideos (9) sämtliche aufeinander folgende Blickpunkte (37, 38, 69, 70), welche jeweils dem ersten Fixationskriterium (25) genügen, zusammen einer ersten Fixation (48) zugeordnet werden, und dass eine erste Fixationsdauer (103) zwischen dem ersten der ersten Fixation (48) zugeordneten Blickpunkt (37) und dem letzten der ersten Fixation (48) zugeordneten Blickpunkt (70) ermittelt wird, und dass die Häufigkeit (56) der ermittelten Fixationen (48, 49) in Abhängigkeit der ersten Fixationsdauer (103) ausgegeben werden, und dass für den ersten Abschnitt des Blickfeldvideos (9) vorzugsweise eine erste Sakkadendauer (104) zwischen dem letzten, der ersten Fixation (48) zugeordneten Blickpunkt (70) und einem ersten, einer zweiten Fixation (49) zugeordneten Blickpunkte (73) ermittelt und insbesondere die Häufigkeit (56) der ermittelten Sakkaden in Abhängigkeit der ersten Sakkadendauer (104) ausgegeben werden.

13. Verfahren zur Messung der Erkennbarkeit vorgebbarer Objekteinheiten, **dadurch gekennzeichnet, dass** für einen vorgebbaren dritten Abschnitt des Blickfeldvideos (9) sämtliche einer vorgebbaren ersten Objekteinheit zuzuordnende Blickpunkte in einem ersten Objektzwischenspeicher (81) gesammelt werden, und dass das Verfahren nach den Ansprüchen 10 bis 12 mit den im ersten Objektzwischenspeicher (81) gesammelten Blickpunkten durchgeführt wird.

14. Verfahren zur Wahmehmungsmessung von vorgebbaren Objekteinheiten, **dadurch gekennzeichnet, dass** das Verfahren nach einem der Ansprüche 1 bis 12 weiters für wenigstens einen vorgebbaren zweiten Abschnitt des Blickfeldvideos (9) mit wenigstens einem vorgebbaren, vom ersten Fixationskriterium (25) unterschiedlichen, zweiten Fixationskriterium (26) durchgeführt wird, und vorzugsweise die Häufigkeit der Fixationen (48, 49) in Ahängigkeit wenigstens des ersten und des zweiten Fixationskriteriums (25, 26) als erste Kurve (58) mit konstanter erster Zeitdauer und als zweite Kurve (59) mit konstanter zweiter Zeitdauer ausgegeben wird.

15. Verfahren zur Überwachung der visuellen Wahrnehmung wenigstens eines ersten, vorzugsweise humanoiden, Benutzers, **dadurch gekennzeichnet, dass** durch wenigstens eine erste Umgebungskamera wenigstens ein erstes Umgebungsvideo der Umgebung des ersten Benutzers aufgenommen wird, dass das erste Umgebungsvideo auf das Vorhandensein wenigstens eines vorgebbaren ersten Musters, vorzugsweise ein Verkehrszeichen, ein untersucht wird, dass mit einem Verfahren nach einem der Ansprüche 1 bis 12 ermittelt wird, ob das erste Fixationskriterium (25) bei mit dem ersten Muster wenigstens bereichsweise deckungsgleichen Blickpunkten erfüllt wird, und dass bei einem Nichterfüllen des ersten Fixationskriteriums bei dem ersten Muster wenigstens eine erste Steuerungs- und/oder Regelungsschaltung aktiviert wird.

## Claims

1. A method for measuring perception, especially for measuring individual visual attention, with at least first sight coordinates of a first point of vision (37), which first sight coordinates are associated to a first field of vision of a field of vision video, and at least second sight coordinates of a second point of vision (38), which second sight coordinates are associated to a second field of vision of the field of vision video, which sight coordinates are especially determined by a vision detection system, with the second field of vision image being recorded subsequently to the first field of vision image, with the second sight coordinates of the second point of vision (38) being checked with the first sight coordinates of the first point of vision (37) in a comparison apparatus for the fulfilment of a least one first predeterminable fixation criterion (25), with the first and second points of vision (37, 38) being allocated and marked upon fulfilment of the first fixation criterion (25) of a first fixation (48) to be allocated to the ordered perception, with the first and second points of vision (37, 38) being allocated and marked upon non-fulfilment of the first fixation criterion (25) to a first saccade to be allocated to random perception, with the field of vision video (9), which is recorded for determining the sight coordinates of the points of vision (37, 38) by the vision detecting system, being output, with the points of vision (37, 38) associated to at least the first fixation (48) or at least the first saccade being displayed in the field of vision video (9).

2. A method according to claim 1, **characterised in that** the first and second points of vision (37, 38) are output as being marked as belonging to the first fixation (48) or first saccade.

3. A method according to claim 1 or 2, **characterized in that** the first fixation criterion (25) can be predetermined, especially that the first fixation criterion (25) is a predeterminable first distance (39) about the first point of vision (37), and that the first relative distance (40) is determined between the first point of vision (37) and the second point of vision (38), and that when the first relative distance (40) is lower than the first distance (39) the first and the second point of vision (37, 38) are allocated to the first fixation (48), with the first distance (39) especially being a first angle of vision (41) which preferably describes an area (34) associated with foveal vision, especially between 0.5° and 1.5°, preferably approx. 1°, and the distance between the first point of vision (37) and the second point of vision (38) is especially a first relative angle (42).

4. A method according to one of the claims 1 to 3, **characterized in that** the second field of vision image was recorded subsequently, and especially directly subsequently, to the first field of vision image after a predeterminable first period oftime, especially between 0.005 s and 0.1 s, preferably between 0.02 s and 0.04 s.

5. A method according to claim 3 or 4, **characterized in that** the first relative distance (40) is output together with points of vision (37, 38) marked as belonging to the first fixation (48) or the first saccade, preferably that the first relative distance (40) is output in a first diagram (11) over the temporal progression of a field of vision video (9).

6. A method according to one of the claims 1 to 5, **characterized in that** a first circle (43) is output in a substantially constant manner about the point of vision (37) with the radius of the first distance (39) together with a point of vision (37) respectively corresponding to the field of vision image currently shown in the field of vision video (9), especially that a second circle (44) is output in a substantially constant manner about the point of vision (37) with the radius of a predeterminable second distance together with a point of vision (37) respectively corresponding to the field of vision image currently shown in the field of vision video (9), which second distance is preferably a second angle of vision which especially describes an area (35) to be associated with parafovael sight, preferably 3° to 5°.

7. A method according to one of the claims 1 to 6, **characterized in that** first traces of vision (45) are determined by connecting successively occurring points of vision (37, 38), which traces of vision are displayed at least temporarily in the field of vision video (9).

8. A method according to one of the claims 1 to 7, **characterized in that** the image points (37) which are marked as belonging to at least the first fixation (48) are displayed to be enclosed in a substantially constant manner by a third circle (46), and that the radius of the third circle (46) is a function of the continuing duration of the first fixation (48).

9. A method according to one of the claims 1 to 8, **characterized in that** the field of vision video (9) is displayed in a dimmed way, that the image points (37) marked as belonging at least to the first fixation (48) are displayed to be enclosed in a substantially constant manner by a fourth circle (47), and that the area of the fourth circle (47) is displayed in a brighter fashion in comparison with the field of vision video (9) displayed in a dimmed way.

10. A method according to one of the claims I to 9, **characterized in that** all successive points of vision (37, 38, 69, 70) which respectively fulfil the first fixation criterion (25) are jointly allocated to a first fixation (48) for a predeterminable first section of the field of vision video (9), and that the angular distance is determined between first the point of vision (37) allocated to the first fixation (48) and the last point of vision (70) allocated to the first fixation (48) and is output as the first fixation angle (51), and that preferably the frequency (56) of the determined fixations (48, 49) is output depending on the fixation angle for the first section of the field of vision video (9).

11. A method according to claim 10, **characterized in that** the angular distance between the last point of vision (70) allocated to the first fixation (48) and a first point of vision (73) allocated to a second fixation (48) is determined for the first section of the field of vision video (9), and that preferably the frequency (56) of the determined saccades is output depending on the saccade angle for the first section of the field of vision video (9).

12. A method according to one of the claims 1 to 11, **characterized in that** all successive points of vision (37, 38, 69, 70) which respectively meet the first fixation criterion (25) are jointly allocated to a first fixation for a predetermined first section of the field of vision video (9), and that a first fixation duration (103) is determined between the first point of vision (37) allocated to the first fixation (48) and the last point of vision (70) allocated to the first fixation (48), and that the frequency (56) of the determined fixations (48, 49) is output depending on the first fixation duration (103), and that preferably a first saccade duration (104) is determined between the last point of vision (70) allocated to the first fixation (48) and a first point of vision (73) allocated to a second fixation (49) for the first section of the field of vision video (9), and especially the frequency (56) of the determined saccades is output depending on the first saccade duration (104).

13. A method for measuring the recognisability of predeterminable object units, **characterized in that** all points of vision to be allocated to a predeterminable first object unit are collected in a first object intermediate storage unit (81) for a predeterminable third section of the field of vision video (9), and that the method according to claims 10 to 12 is performed with the points of vision collected in the first object intermediate storage unit (81).

14. A method for measuring the perception of predeterminable object units, **characterized in that** the method according to one of the claims 1 to 12 is further performed for at least one predeterminable second section of the field of vision video (9) with at least one predeterminable second fixation criterion (26) which differs from the first fixation criterion (25), and preferably the frequency of the fixations (48, 49) is output depending on at least the first and second fixation criterion (25, 26) as a first curve (58) of constant first duration and as second curve (59) of constant second duration.

15. A method for monitoring the visual perception of at least one first, preferably humanoid, user, **characterized in that** at least one first ambient video of the ambient environment of the first user is recorded by at least one first ambient camera, that the first ambient video is examined for the presence of at least one predeterminable first pattern, preferably a traffic sign, that by using a method according to one of the claims I to 12 it is determined whether the first fixation criterion (25) is fulfilled with points of vision which are congruent at least in part with the first pattern, and that in the case of non-fulfilment of the first fixation criterion in the first pattern at least one first open-loop and/or closed-loop control circuit will be activated.

## Revendications

1. Procédé pour la mesure de la perception, en particulier pour la mesure de l'attention visuelle individuelle, dans lequel au moins des premières coordonnées visuelles d'un premier point regardé (37) associées à une première image de champ visuel d'une vidéo de champ visuel et au moins des deuxièmes coordonnées visuelles d'un deuxième point regardé (38) associées à une deuxième image de champ visuel de la vidéo de champ visuel, déterminées en particulier par un système de détection du regard, sont traitées, dans lequel la deuxième image de champ visuel est acquise après la première image de champ visuel, dans lequel les deuxièmes coordonnées visuelles du deuxième point regardé (38) sont vérifiées avec les premières coordonnées visuelles du premier point regardé (37) dans un dispositif de comparaison pour vérifier qu'au moins un premier critère de fixation (25) pouvant être prédéterminé est satisfait, dans lequel les premier et deuxième points regardés (37, 38) sont associés, si le premier critère de fixation (25) est satisfait, à une première fixation (48) à associer à la perception ordonnée et caractérisés, dans lequel les premier et deuxième points regardés (37, 38) sont associés, si le premier critère de fixation (25) n'est pas satisfait, à une première saccade à associer à une perception désordonnée et caractérisés, dans lequel la vidéo de champ visuel (9) acquise par le système de détection du regard est émise en sortie pour déterminer les coordonnées visuelles des points regardés (37, 38), dans lequel les points regardés (37, 38) correspondant au moins à la première fixation (48) ou au moins à la première saccade sont représentés dans la vidéo de champ visuel (9).

2. Procédé selon la revendication 1, **caractérisé en ce que** les premier et deuxièmes points regardés (37, 38) sont émis en sortie en étant caractérisés comme associés à la première fixation (48) ou à la première saccade.

3. Procédé selon la revendication 1 ou 2, **caractérisé en ce que** le premier critère de fixation (25) peut être prédéterminé, en particulier **en ce que** le premier critère de fixation (25) est une première distance (39) pouvant être prédéterminée autour du premier point regardé (37), et **en ce que** la première distance relative (40) entre le premier point regardé (37) et le deuxième point regardé (38) est déterminée, et **en ce que** lorsque la première distance relative (40) est plus petite que la première distance (39), le premier point regardé et le deuxième (37, 38) sont associés à la première fixation (48), la première distance (39) étant en particulier un premier angle de regard (41) qui décrit en particulier une zone (34) associée à la vision fovéale, en particulier entre 0,5° et 1,5°, de préférence à environ 1°, et la distance entre le premier point regardé (37) et le deuxième point regardé (38) est en particulier un premier angle relatif (42).

4. Procédé selon l'une des revendications 1 à 3, **caractérisé en ce que** la deuxième image de champ visuel est acquise après un premier laps de temps pouvant être prédéterminé, en particulier de 0,005 s à 0,1 s, de préférence de 0,02 s à 0,04 s, après la première image de champ visuel, en particulier immédiatement après.

5. Procédé selon la revendication 3 ou 4, **caractérisé en ce que** la première distance relative (40) est émise en sortie avec les points regardés (37, 38) caractérisés comme associés à la première fixation (48) ou à la première saccade, de préférence **en ce que** la première distance relative (40) est émise en sortie dans un premier diagramme (11) sur le déroulement dans le temps d'une vidéo de champ visuel (9).

6. Procédé selon l'une des revendications 1 à 5, **caractérisé en ce qu'**en même temps que le point regardé (37) correspondant à l'image du champ visuel actuellement représentée dans la vidéo de champ visuel (9), un premier cercle (43) ayant le rayon de la première distance (39) est émis de façon sensiblement régulière autour du point regardé (37), en particulier **en ce qu'**en même temps qu'un point regardé (37) correspondant à l'image du champ visuel actuellement représentée dans la vidéo de champ visuel (9), un deuxième cercle (44) ayant le rayon d'une deuxième distance pouvant être prédéterminée est émis de façon sensiblement régulière autour du point regardé (37), laquelle deuxième distance est de préférence un deuxième angle de regard qui décrit en particulier une zone (35) associée à la vision parafovéale, de préférence de 3° à 5°.

7. Procédé selon l'une des revendications 1 à 6, **caractérisé en ce que** l'association de points regardés successifs (37, 38) détermine des premières traces de regard (45) qui sont représentées au moins temporairement dans la vidéo de champ visuel (9).

8. Procédé selon l'une des revendications 1 à 7, **caractérisé en ce que** les points regardés (37) caractérisés comme associés au moins à la première fixation (48) sont représentés entourés d'un troisième cercle (46) sensiblement régulier et **en ce que** le rayon du troisième cercle (46) est une fonction de la durée continue dans le temps de la première fixation (48).

9. Procédé selon l'une des revendications 1 à 8, **caractérisé en ce que** la vidéo de champ visuel (9) est représentée assombrie, **en ce que** les points regardés (37) caractérisés comme associés au moins à la première fixation (48) sont entourés de façon sensiblement régulière par un quatrième cercle (47) et **en ce que** l'aire du quatrième cercle (47) est représentée au moins temporairement plus claire que la vidéo de champ visuel (9) représentée assombrie.

10. Procédé selon l'une des revendications 1 à 9, **caractérisé en ce que** pour une première section pouvant être prédéterminée de la vidéo de champ visuel (9), tous les points regardés successifs (37, 38, 69, 70) satisfaisant chacun le premier critère de fixation (25) sont associés ensemble à une première fixation (48), et **en ce que** l'écart angulaire entre le premier point regardé (37) associé à la première fixation (48) et le dernier point regardé (70) associé à la première fixation (48) est déterminé et émis en sortie comme premier angle de fixation (51), et **en ce que** pour la première section de la vidéo de champ visuel (9), la fréquence (56) des fixations (48, 49) déterminées est de préférence émise en sortie en fonction de l'angle de fixation.

11. Procédé selon la revendication 10, **caractérisé en ce que** pour la première section de la vidéo de champ visuel (9), l'écart angulaire entre le dernier point regardé (70) associé à la première fixation (48) et un premier point regardé (73) associé à une deuxième fixation (49) est déterminé et émis en sortie comme premier angle de saccade (52), et **en ce que** pour la première section de la vidéo de champ visuel (9), la fréquence (56) des saccades déterminées est de préférence émise en sortie en fonction de l'angle de saccade.

12. Procédé selon l'une des revendications 1 à 11, **caractérisé en ce que** pour une première section pouvant être prédéterminées de la vidéo de champ visuel (9), tous les points regardés successifs (37, 38, 69, 70) satisfaisant chacun le premier critère de fixation (25) sont associés ensemble à une première fixation (48) et **en ce qu'**une première durée de fixation (103) entre le premier point regardé (37) associé à la première fixation (48) et le dernier point regardé (70) associé à la première fixation (48) est déterminée, et **en ce que** la fréquence (56) des fixations (48, 49) déterminées est émise en sortie en fonction de la première durée de fixation (103), et **en ce que** pour la première section de la vidéo de champ visuel (9), une première durée de saccade (104) entre le dernier point regardé (70) associé à la première fixation (48) et un premier point regardé (73) associé à une deuxième fixation (49) est déterminée, et la fréquence (56) des saccades déterminées est en particulier émise en sortie en fonction de la première durée de saccade(104).

13. Procédé de mesure de la possibilité de reconnaissance d'unités d'objets pouvant être prédéterminées, **caractérisé en ce que** pour une troisième section pouvant être prédéterminée de la vidéo de champ visuel (9), tous les points regardés à associer à une première unité d'objet pouvant être prédéterminée sont rassemblés dans une première mémoire tampon d'objets (81) et **en ce que** le procédé est exécuté selon les revendications 10 à 12 avec les points regardés rassemblés dans la première mémoire tampon d'objets (81).

14. Procédé pour la mesure de la perception d'unités d'objet pouvant être prédéterminées, **caractérisé en ce que** le procédé selon l'une des revendications 1 à 12 est exécuté en outre pour au moins une deuxième section pouvant être prédéterminée de la vidéo de champ visuel (9) avec au moins un deuxième critère de fixation (26) pouvant être prédéterminé différent du premier critère de fixation (25), et la fréquence des fixations (48, 49) est de préférence émise en sortie en fonction au moins du premier critère de fixation et du deuxième (25, 26) sous la forme d'une courbe fixe (58) ayant une première durée dans le temps constante et d'une deuxième courbe (59) ayant une deuxième durée dans le temps constante.

15. Procédé pour la surveillance de la perception visuelle d'au moins un premier utilisateur, de préférence humanoïde, **caractérisé en ce qu'**au moins une première caméra d'environnement acquiert au moins une première vidéo d'environnement du premier utilisateur, **en ce que** la première vidéo d'environnement est analysée pour déterminer la présence d'au moins un premier motif pouvant être prédéterminé, de préférence un signal de circulation, en que qu'un procédé selon l'une des revendications 1 à 12 est mis en couvre pour déterminer si le premier critère de fixation (25) est satisfait au moins par des points regardés ayant par endroits la même étendue que le premier motif, et **en ce que** si le premier critère de fixation n'est pas rempli avec le premier motif, au moins un circuit de commande et/ou de régulation est activé.
